# NOUVEAU FASCICULE DE BREVET EUROPEEN

(11) **EP 1 329 216 B2**
(45) Date de publication et mention de la décision concernant l'opposition: **06.07.2016**
(45) Mention de la délivrance du brevet: 16.07.2008
(21) Numéro de dépôt: 02293200.8
(22) Date de dépôt: 20.12.2002
(51) Int. Cl.: A61K 8/39, A61Q 5/10

(54) **Composition pour la teinture d'oxydation des fibres kératiniques comprenant un acide éther carboxylique oxyalkyléné, un tensioactif non-ionique et un polymère particulier**
Oxidationsfärbemittel für keratinische Fasern enthaltend eine Oxyalkylenäthercarbonsäure, ein nicht-ionisches Tensid und ein Polymer
Composition for oxidative dyeing of keratinous fibers containing an oxyalkylene ether carboxylic acid, a non-ionic surfactant and a particulate polymer

(30) Priorité: 21.12.2001 FR 0116738
(43) Date de publication de la demande: 23.07.2003
(73) Titulaire: L'Oréal, 75008 Paris (FR)
(72) Inventeur: Desenne, Patricia, 92300 Levallois Perret (FR); Bebot, Cécile, 92110 Clichy (FR); Laurent, Florence, 92270 Bois-Colombes (FR)
(74) Mandataire: Wattremez, Catherine

(56) Documents cités:
- EP-A- 0 188 216
- EP-A- 0 733 355
- EP-A1- 1 036 557
- EP-A1- 1 142 556
- WO-A1-94/24097
- DE-A- 3 044 738
- DE-A1- 3 929 973
- DE-A1- 4 227 864
- DE-A1- 10 016 279
- DE-A1- 10 027 975
- DE-A1- 19 959 320
- DE-A1- 19 962 882
- FR-A- 2 680 682
- FR-A- 2 714 289
- GB-A- 2 066 663
- US-A1- 2001 023 514
- J. FALBE ET AL.: 'Römpp, Chemie Lexikon', vol. 3, 1995, THIEME VERLAG, STUTTGART page 2246

## Description

La présente invention concerne une composition pour la teinture d'oxydation des fibres kératiniques, en particulier des fibres kératiniques humaines et plus particulièrement les cheveux, comprenant, dans un milieu approprié pour la teinture au moins un colorant d'oxydation, et en outre au moins un acide éther carboxylique polyoxyalkyléné ou l'un de ses sels, au moins un agent tensioactif non-ionique particulier et au moins un polymère cationique ou amphotère particulier dont la densité de charge cationique est supérieure ou égale à 2 meq/gramme.
L'invention concerne également les procédés et dispositifs de teinture mettant en oeuvre ladite composition.

Il est connu de teindre les fibres kératiniques et en particulier les cheveux humains, avec des compositions de teinture contenant des.précurseurs de colorants d'oxydation, généralement appelés "bases d'oxydation", en particulier des ortho- ou paraphénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

Ces bases d'oxydation et ces coupleurs sont formulés dans des véhicules ou supports permettant leur application sur les fibres kératiniques après mélange avec un agent oxydant.
Ces véhicules sont généralement aqueux et comportent usuellement un ou plusieurs agents tensioactifs.

Ainsi, on a déjà préconisé d'utiliser des acides gras à titre d'agent tensioactif anionique, en association avec un polymère cationique ou amphotère et un agent tensioactif non-ionique. Lesdites associations permettent d'obtenir des compositions pour la teinture d'oxydation qui engendrent des nuances ayant de très bonnes propriétés tinctoriales. Cependant, on continue à rechercher des supports améliorant encore les propriétés des colorations obtenues.

On connaît également d'après le document EP 733 355, des shampooings colorants ayant des propriétés tinctoriales satisfaisantes, comprenant au moins un colorant direct et au moins un tensioactif anionique choisi parmi des acides alkyl amidoéther carboxyliques particuliers.

Le document FR 2 714 289 décrit également des compositions cosmétiques comprenant un mélange de polymères cationiques conditionneurs particuliers. Ces compositions, qui permettent d'améliorer la douceur de la peau ou le démêlage des cheveux, peuvent également être mises en oeuvre dans le domaine de la coloration.

Ainsi, après d'importantes recherches menées sur la question, la Demanderesse vient maintenant de découvrir qu'il est possible d'obtenir des compositions de teinture d'oxydation qui permettent d'obtenir des nuances encore plus puissantes et plus chromatiques, en introduisant dans la composition tinctoriale, au moins un acide éther carboxylique polyoxyalkyléné ou l'un de ses sels de formule (I) suivante : 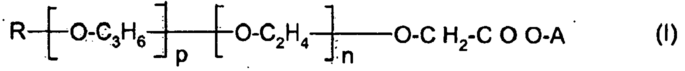 dans laquelle :
R représente un radical ou un mélange de radicaux alkyle ou alcényle linéaire ou ramifié en C8-C22,
n est un nombre entier ou décimal allant de 2 à 24,
p est un nombre entier ou décimal allant de 0 à 6,
A désigne un atome d'hydrogène ou bien Na, K, Li, 1/2Mg ou un reste monoéthanolamine, ammonium ou triéthanolamine ; et
au moins un agent tensioactif non-ionique choisis dans le groupe formé par :
1) les alcools gras comportant de 8 à 22 atomes de carbone et oxyéthylénés par 1 à 10 moles d'oxyde d'éthylène.
2) les alcools gras mono- ou poly-glycérolés de formule (II) suivante : 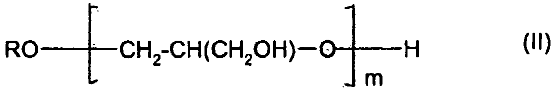 dans laquelle :
   R représente un radical saturé ou insaturé, linéaire ou ramifié, comportant de 8 à 40 atomes de carbone et de préférence de 10 à 30 atomes de carbone ;
   m représente un nombre allant de 1 à 30 et de préférence de 1 à 10 ;
et au moins un polymère cationique ou amphotère dont la densité de charge cationique est supérieure ou égale à 2 meq/gramme.

Cette découverte est à la base de la présente invention.

La présente invention a ainsi pour objet une composition pour la teinture d'oxydation des fibres kératiniques, en particulier des fibres kératiniques humaines, et plus particulièrement les cheveux, comprenant, dans un milieu approprié pour la teinture, au moins un colorant d'oxydation, et caractérisée par le fait qu'elle comprend en outre au moins un acide éther carboxylique polyoxyalkyléné de formule (I) détaillée ci-après ou l'un de ses sels, au moins un agent tensioactif non-ionique et au moins un polymère cationique ou amphotère dont la densité de charge cationique est supérieure ou égale à 2 meq/gramme, dans laquelle
- les acides éther carboxyliques polyoxyalkylénés ou leurs sels sont de formule (I) suivante : 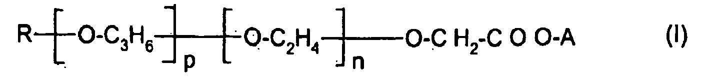 dans laquelle :
   R représente un radical ou un mélange de radicaux alkyle ou alcényle linéaire ou
   ramifié en C8-C22,
   n est un nombre entier ou décimal allant de 2 à 24,
   p est un nombre entier ou décimal allant de 0 à 6,
   A désigne un atome d'hydrogène ou bien Na, K, Li, 1/2Mg ou un reste monoéthanolamine, ammonium ou triéthanolamine,
      - les tensioactif non ioniques sont choisis dans le groupe formé par :
         1) les alcools gras comportant de 8 à 22 atomes de carbone et oxyéthylénés par 1 à 10 moles d'oxyde d'éthylène.
         2) les alcools gras mono- ou poly-glycérolés de formule (II) suivante : 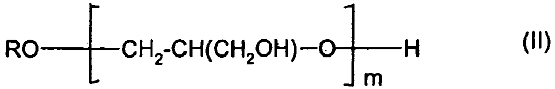 dans laquelle :
            R représente un radical saturé ou insaturé, linéaire ou ramifié, comportant de 8 à 40 atomes de carbone et de préférence de 10 à 30 atomes de carbone ;
            m représente un nombre allant de 1 à 30 et de préférence de 1 à 10.

Un autre objet de l'invention porte sur une composition prête à l'emploi pour la teinture d'oxydation des fibres kératiniques qui comprend, dans un milieu approprié pour la teinture, au moins une composition telle que décrite ci-avant et au moins un agent oxydant.
Par composition prête à l'emploi, on entend au sens de la présente invention, la composition destinée à être appliquée immédiatement sur les fibres kératiniques, c'est à dire qu'elle peut être stockée telle quelle avant utilisation ou résulter du mélange extemporané de deux ou plusieurs compositions.
L'invention vise également un procédé de teinture d'oxydation des fibres kératiniques, et en particulier des fibres kératiniques humaines telles que les cheveux, consistant à appliquer sur les fibres une composition colorante comprenant, dans un milieu approprié pour la teinture, au moins un colorant d'oxydation, la couleur étant révélée à pH alcalin, neutre ou acide à l'aide d'une composition oxydante comprenant, dans un milieu approprié pour la teinture, au moins un agent oxydant, qui est mélangée juste au moment de l'emploi à la composition colorante ou qui est appliquée séquentiellement sans rinçage intermédiaire, la composition colorante et la composition oxydante comprenant en outre et répartis indifféremment entre elles-deux, au moins un acide éther carboxylique polyoxyalkyléné de formule (I) détaillée ci-après ou l'un de ses sels, au moins un agent tensioactif non-ionique et au moins un polymère cationique ou amphotère dont la densité de charge cationique est supérieure ou égale à 2 meq/gramme, dans laquelle
- les acides éther carboxyliques polyoxyalkylénés ou leurs sels sont de formule (I) suivante : 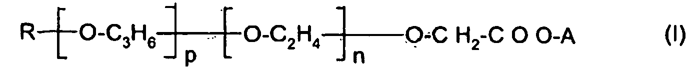 dans laquelle :
   R représente un radical ou un mélange de radicaux alkyle ou alcényle linéaire ou ramifié en C8-C22,
   n est un nombre entier ou décimal allant de 2 à 24,
   p est un nombre entier ou décimal allant de 0 à 6,
   A désigne un atome d'hydrogène ou bien Na, K, Li, 1/2Mg ou un reste monoéthanolamine, ammonium ou triéthanolamine,

- les tensioactifs non ioniques sont choisis dans le groupe formé par :
   1) les alcools gras comportant de 8 à 22 atomes de carbone et oxyéthylénés par 1 à 10 moles d'oxyde d'éthylène.
   2) les alcools gras mono- ou poly-glycérolés de formule (II) suivante : 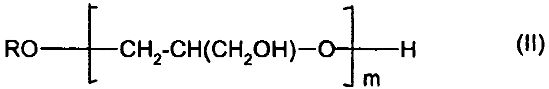 dans laquelle :
      R représente un radical saturé ou insaturé, linéaire ou ramifié, comportant de 8 à 40 atomes de carbone et de préférence de 10 à 30 atomes de carbone ;
      m représente un nombre allant de 1 à 30 et de préférence de 1 à 10.
Dans une variante dudit procédé, le ou les acide(s) éther carboxylique polyoxyalkyléné(s) de formule (I) ou leurs sels, agent(s) tensioactif(s) non-ionique(s) et polymère(s) cationique(s) ou amphotère(s) dont la densité de charge cationique est supérieure ou égale à 2 meq/gramme sont réunis dans la même composition colorante ou oxydante et plus particulièrement encore dans la composition colorante.

L'invention a également pour objet un dispositif de teinture à plusieurs compartiments ou " kit " pour la teinture d'oxydation des fibres kératiniques, en particulier des fibres kératiniques humaines et plus particulièrement les cheveux qui comporte un compartiment renfermant une composition colorante comprenant, dans un milieu approprié pour la teinture, au moins un colorant d'oxydation, et un autre compartiment renfermant une composition oxydante comprenant, dans un milieu approprié pour la teinture, un agent oxydant, la composition colorante et la composition oxydante comprenant en outre et répartis indifféremment entre elles-deux, au moins un acide éther carboxylique polyoxyalkyléné de formule (I), au moins un agent tensioactif non-ionique et au moins un polymère cationique ou amphotère dont la densité de charge est supérieure ou égale à 2 meq/gramme, dans laquelle -les acides éther carboxyliques polyoxyalkylénés ou leurs sels sont de formule (I) suivante : 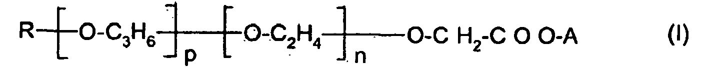 dans laquelle :
R représente un radical ou un mélange de radicaux alkyle ou alcényle linéaire ou ramifié en C8-C22,
n est un nombre entier ou décimal allant de 2 à 24,
p est un nombre entier ou décimal allant de 0 à 6,
A désigne un atome d'hydrogène ou bien Na, K, Li, 1/2Mg ou un reste monoéthanolamine, ammonium ou triéthanolamine,
   - les tensioactifs non ioniques sont choisis dans le groupe formé par :
      1) les alcools gras comportant de 8 à 22 atomes de carbone et oxyéthylénés par 1 à 10 moles d'oxyde d'éthylène.
      2) les alcools gras mono- ou poly-glycérolés de formule (II) suivante : 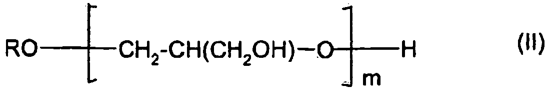 dans laquelle :
         R représente un radical saturé ou insaturé, linéaire ou ramifié, comportant de 8 à 40 atomes de carbone et de préférence de 10 à 30 atomes de carbone ;
         m représente un nombre allant de 1à 30 et de préférence de 1 à 10.
Dans une variante de dispositif, le ou les acide(s) éther carboxylique polyoxyalkyiéné(s) de formule (I) ou leurs sels, agent(s) tensioactif(s) non-ionique(s) et polymère(s) cationique(s) ou amphotère(s) dont la densité de charge cationique est supérieure ou égale à 2 meq/gramme sont réunis dans la même composition colorante ou oxydante et plus particulièrement encore dans la composition colorante.

Mais d'autres caractéristiques, aspects, objets et avantages de l'invention apparaîtront encore plus clairement à la lecture de la description et des exemples qui suivent.

### Acides éther carboxylique oxyalkylénés et leurs sels

Par acide éther carboxylique oxyalkyléné, on entend tout composé présentant la formule (I) suivante : 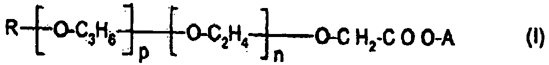 dans laquelle :
R représente un radical ou un mélange de radicaux alkyle ou alcényle linéaire ou ramifié en C₈-C₂₂.
n est un nombre entier ou décimal allant de 2 à 24,
p est un nombre entier ou décimal allant de 0 à 6,
A désigne un atome d'hydrogène ou bien Na, K, Li, 1/2Mg ou un reste monoéthanolamine, ammonium ou triéthanolamine.
Les acides éther carboxylique oxyalkylénés ou leurs sels utilisés de préférence selon la présente invention sont choisis parmi ceux de formule (I) dans laquelle R désigne un radical ou un mélange de radicaux alkyl(C₁₂-C₁₄), oléyle, cétyle, stéaryle ; A désigne un atome d'hydrogène ou de sodium, p=0, et n varie de 2 à 20 et de préférence 2 à 10.
Plus préférentiellement encore, on utilise des composés de formule (I) dans laquelle R désigne un radical alkyl(C₁₂), A désigne un atome d'hydrogène ou de sodium, p=0, et n varie de 2 à 10.

Parmi les produits commerciaux, on peut utiliser de préférence les produits vendus par la société CHEM Y sous les dénominations :
AKYPO® RLM 38 (R= alkyl(C₁₂-C₁₄), n=3,8, p=0, A=H)
AKYPO® RLM 38 NV (R= alkyl(C₁₂-C₁₄), n=4, p=0, A=Na)
AKYPO® RLM 45 (R= alkyl(C₁₂-C₁₄), =4,5, p=0, A=H)
AKYPO® RLM 45 NV (R= alkyl(C₁₂-C₁₄), n=4,5, p=0, A=Na)
AKYPO® RLM 100 (R= alkyl(C₁₂-C₁₄), n=10, p=0, A=H)
AKYPO® RLM 100 NV (R= alkyl(C₁₂-C₁₄), n=10, p=0, A=Na)
AKYPO® RLM 130 (R= alkyl(C₁₂-C₁₄), n=13, p=0, A=H)
AKYPO® RLM 160 NV (R= alkyl(C₁₂-C₁₄), n=16, p=0, A=Na)
AKYPO® RO 20 (R=oléyle, n=2, p=0, A=H)
AKYPO® RO 90 (R=oléyle, n=9, p=0, A=H)
AKYPO® RCS 60 (R=cétyle/stéaryle, n=6, p=0, A=H)
AKYPO® RS 60 (R=stéaryle, n=6, p=0, A=H)
AKYPO® RS 100 (R=stéaryle, n=10, p=0, A=H)
AKYPO® RO 50 (R=oléyle, n=5, p=0, A=H), ou par la société SANDOZ sous les dénominations :
SANDOPAN ACA-48 (R=cétyle/stéaryle, n=24, p=0, A=H)
SANDOPAN DTC-Acid (R= alkyl(C₁₃), n=6, p=0, A=H)
SANDOPAN DTC (R= alkyl(C₁₃), n=6, p=0, A=Na)
SANDOPAN LS 24 (R= alkyl(C₁₂-C₁₄), n=12, p=0, A=Na)
SANDOPAN JA 36 (R= alkyl(C₁₃), n=18, p=0, A=H),
et plus particulièrement, les produits vendus sous les dénominations suivantes :
AKYPO® RLM 25
AKYPO® RLM 45
AKYPO® RLM 100
AKYPO® RO 20
AKYPO® RO 50
AKYPO® RLM 38.

Les acides éther carboxyliques oxyalkylénés ou leurs sels représentent environ de 2 à 15%, et de préférence environ de 3 à 10% du poids total de la composition de teinture et d'environ 0,5 à 15% et de préférence d'environ 0,7 à 10% du poids total de celle prête à l'emploi (comprenant l'oxydant).

### Tensioactifs non ioniques

Les agents tensioactifs non-ioniques sont des composés bien connus en soi (voir notamment à cet égard "Handbook of Surfactants" par M.R. PORTER, éditions Blackie & Son (Glasgow and London), 1991, pp 116-178).

Selon l'invention, on utilise :
1. 1) les alcools gras comportant de 8 à 22 atomes de carbone et oxyéthylénés par 1 à 10 moles d'oxyde d'éthylène (1 à 10 OE). Parmi eux, on peut citer plus particulièrement l'alcool laurique 2 OE, l'alcool laurique 3 OE, l'alcool décylique 3 OE et l'alcool décylique 5 OE.
2. 2) les alcools gras mono- ou poly-glycérolés que l'on peut représenter par la formule (II) suivante : 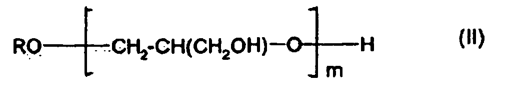 dans laquelle :
   R représente un radical saturé ou insaturé, linéaire ou ramifié, comportant de 8 à 40 atomes de carbone et de préférence de 10 à 30 atomes de carbone ;
   m représente un nombre allant de 1 à 30 et de préférence de 1 à 10.

Comme composés de ce type, on peut citer, l'alcool laurique à 4 moles de glycérol (nom INCl : POLYGLYCERYL-4 LAURYL ETHER), Palcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCl : POLYGLYCERYL-2 OLEYL ETHER), l'alcool cétéarylique à 2 moles de glycérol, l'alcool cétéarylique à 6 moles de glycérol , l'alcool oléocétylique à 6 moles de glycérol, et l'octadécanol à 6 moles de glycérol.
L'alcool gras peut représenter un mélange d'alcools gras au même titre que la valeur de m représente une valeur statistique, ce qui signifie que dans un produit commercial peuvent coexister plusieurs espèces d'alcools gras polyglycérolés sous forme d'un mélange.
Parmi les alcools gras mono- ou poly-glycérolés, on préfère plus particulièrement utiliser l'alcool en C8/C10 à une mole de glycérol, l'alcool en C10/C12 à 1 mole de glycérol et l'alcool en C12 à 1,5 moles de glycérol.

Le ou les tensioactifs non-ioniques représentent environ de 2 à 40%, et de préférence environ de 4 à 20% du poids total de la composition de teinture et d'environ 0,5 à 40% et de préférence d'environ 1 à 20% du poids total de celle prête à l'emploi (comprenant l'oxydant).

### Polymères cationiques ou amphotères

Les polymères cationiques ou amphotères selon la présente invention doivent présenter une densité de charge cationique supérieure ou égale à 2 meq par gramme.
La densité de charge peut être déterminée selon la méthode de Kjeldahl. Elle est généralement mesurée à un pH de 3,9.
La méthode Kjeldahl est bien connue de l'homme de l'art et par exemple décrite, pour le micro Kjeldahl dans :
AACC: approved methods of the American Association of Cereal Chemists, 9 th ed., St Paul, MN (1995),
AOAC: official methods of analysis of AOAC International, 16 th ed., Arlington, VI (1995), et pour le semimicro Kjeldahl dans :
   Vogel A.l: elementary practical organic chemistry Part 3 "quantitative Organic Analysis", Longman Group.

Les polymères cationiques de densité de charge cationique supérieure ou égale à 2 meq par gramme peuvent être choisis de préférence dans le groupe formé par :
(1) les polymères quaternaires de vinylpyrrolidone et de vinylimidazole tels que par exemple les produits commercialisés sous les dénominations Luviquat FC 905®, FC 550® et FC 370® par la société B.A.S.F.
(2) les homopolymères et copolymères comportant dans la chaîne des motifs de formule (III) suivante : 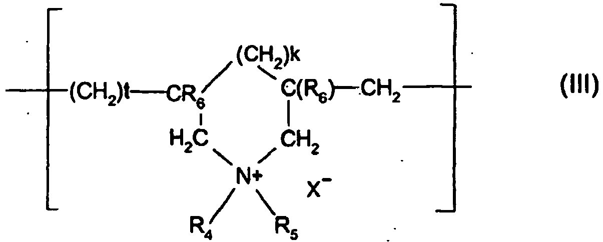 dans laquelle k et t sont égaux à 0 ou 1, la somme k + t étant égaie à 1 ;
   R₄ et R₅, identiques ou différents, désignent un groupement alkyle ayant de 1 à 8 atomes de carbone, un groupement hydroxyalkyle(C₁-C₅), un groupement amidoalkyle(C₁-C₄), ou R₄ et R₅ désignent conjointement avec l'atome d'azote auquel Ils sont rattachés, un groupement pipéridinyle ou morpholinyle ;
   R₆ désigne un atome d'hydrogène ou un radical méthyle ;
   X- est un anion.
   De préférence, R₄ et R₅ identiques ou différents désignent un groupement alkyle ayant de 1 à 4 atomes de carbone ;
   X-est un anion bromure, chlorure, acétate, borate, citrate, tartrate, bisulfate, bisulfite, sulfate, phosphate.
   De tels polymères sont notamment décrits dans le brevet français 2.080.759 et dans son certificat d'addition 2.190.406.
   On peut citer plus particulièrement parmi eux, l'homopolymère de chlorure de diméthyldiallylammonium (POLY-QUATERNIUM 6) vendu sous la dénomination "Merquat®100" par la société Calgon et ses homologues de faible masse moléculaire moyenne en poids et les copolymères de chlorure de diméthyldiallylammonium et d'acrylamide commercialisés sous la dénomination "MEROUAT® 550".
(3) les polymères de diammonium quaternaire contenant des motifs récurrents de formule (IV) suivante : 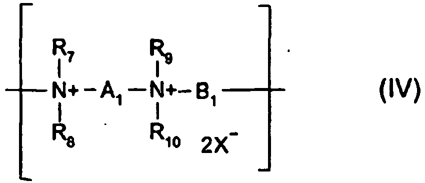 dans laquelle :
   R₇, R₈, R₉ et R₁₀, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₇, R₈, R₉ et R₁₀, ensemble ou séparément, constituent avec les atomes d'azote auxquels ils sont rattachés des hétérocycles contenant éventuellement un second hétéroatome autre que l'azote,
      ou bien R₇, R₈, R₉ et R₁₀, représentent un radical alkyle en C₁-C₆ linéaire ou ramifié substitué par un groupement nitrile, ester, acyle, amide, ou -CO-O- R₁₁-D, ou -CO-NH-R₁₁-D dans lesquels R₁₁ est un alkylène et D un groupement ammonium quaternaire ;
   A₁ et B₁ représentent des groupements polyméthyléniques contenant de 2 à 20 atomes de carbone, linéaires ou ramifiés, saturés ou insaturés, et pouvant contenir, liés à ou Intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X- est un anion;
   A₁, R₇ et R₉ peuvent former avec les deux atomes d'azote auxquels ils sont rattachés, un cycle pipérazinique ; en outre si A, désigne un radical alkylène ou hydroxyalkylène linéaire ou ramifié, saturé ou insaturé, B₁ peut également désigner un groupement - (CH₂)n-CO-D-OC-(CH₂)n- dans lequel n est compris entre 1 et 100 et de préférence entre 1 et 50, et D désigne :
      a) un reste de glycol de formule : -O-Z-O**-,** où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule: -NH-γ-NH-, où Y désigne un radical hydrocarboné linéaire ou ramifié, ou bien le radical bivalent

         -CH₂-CH₂ S-S-CH₂-CH₂- ;
      d) un groupement uréylène de formule : -NH-CO-NH-.

Les polymères cationiques comportant des motifs de formule (IV) ont une masse moléculaire moyenne en nombre généralement comprise entre 1000 et 100000.
Dans ces polymères, X- est un anion chlorure ou bromure.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462,2.261.002, 2.271.378, 3.874.870, 4.001.432, 3.929.990, 3.966.904, 4.005.193, 4.025.617, 4.025.627, 4.025.653, 4.026.945 et 4.027.020.
On peut utiliser plus particulièrement parmi eux, les polymères qui sont constitués de motifs récurrents répondant à la formule (IV)bis suivante: 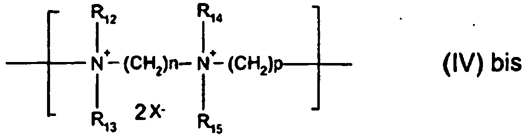 dans laquelle R₁₂, R₁₃, R₁₄ et R₁₅, identiques ou différents, désignent un radical alkyle ou hydroxyalkyle ayant de 1 à 4 atomes de carbone environ, n et p sont des nombres entiers variant de 2 à 20.
Encore plus particulièrement, on préfère les composés de formule (VI) dans laquelle R₁₂, R₁₃, R₁₄ et R₁₅ représentent un radical méthyle ou éthyle.
Des polymères cationiques de formule (VI) particulièrement préférés sont ceux pour lesquels R₁₂, R₁₃, R₁₄ et R₁₅ représentent un radical méthyle et n = 3, p = 6 et X = C1, et notamment ceux dont le poids moléculaire, déterminé par chromatographie par perméation de gel, est compris entre 9500 et 9900 [Polymère W].
D'autres polymères cationiques de formule (VI) particulièrement préférés sont ceux pour lesquels R₁₂ et R₁₃ représentent un radical méthyle, R₁₄ et R₁₅ représentent un radical éthyle et n = p = 3 et X = Br, et notamment ceux dont le poids moléculaire, déterminé par chromatographie par perméation de gel, est d'environ 1200 [Polymère U].

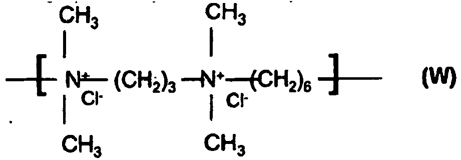

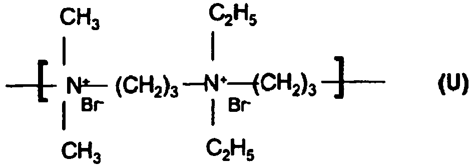

Lesdits polymères à motifs (W) et (U) sont préparés et décrits dans le brevet français 2 270 846.
(4) les polymères de diammonium quaternaire constitués de motifs de formule (V) suivante: 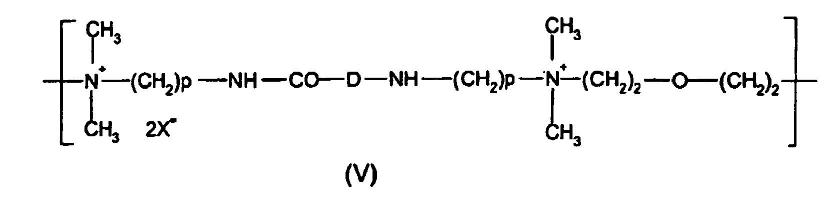 dans laquelle :
   p désigne un nombre entier variant de 1 à 6 environ,
   D peut être nul ou peut représenter un groupement -(CH₂),-CO-dans lequel r désigne un nombre égal à 4 ou à 7, et
   X- est un anion.

Les polymères cationiques comportant des motifs de formule (V) sont notamment décrits dans la demande de brevet EP-A-122 324.

Parmi ces polymères, on préfère ceux de masse moléculaire mesurée par RMN du Carbone 13 inférieure à 100000, et dans la formule de laquelle :
p désigne un nombre entier variant de 1 à 6 environ,
D peut être nul ou peut représenter un groupement-(CH₂)ᵣ-CO-dans lequel r désigne un nombre égal à 4 ou à 7, et X- est un anion dérivé d'un acide minéral ou organique.

Parmi lesdits polymères à motifs de formule (V), on préfère encore ceux pour lesquels, p est égal à 3, et,
a) D représente un groupement -(CH₂)₄-CO-, X désigne un atome de chlore, la masse moléculaire mesurée par RMN du Carbone 13 (RMN¹³C) étant d'environ 5600 ; un polymère de ce type est proposé par la société MIRANOL sous le nom de MIRAPOL-AD1,
b) D représente un groupement-(CH₂)-CO-, X désigne un atome de chlore, la masse moléculaire mesurée par RMN du Carbone 13 (RMN¹³C) étant d'environ 8100 ; un polymère de ce type est proposé par la société MIRANOL sous le nom de MIRAPOL-AZ1,
c) D désigne la valeur zéro, X désigne un atome de chlore, la masse moléculaire mesurée par RMN du Carbone 13, (RMN¹²C) étant d'environ 25500 ; un polymère de ce type est vendu par la société MIRANOL sous le nom MIRAPOL-A15,
d) un "Block Copolymer" formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500). Plus particulièrement encore, on préfère selon l'invention le polymère à motifs de formule (V) dans laquelle p est égal à 3, D désigne la valeur zéro, X désigne un atome de chlore, la masse moléculaire mesurée par RMN du Carbone 13, (RMN¹³C) étant d'environ 25500.

Lesdits polymères cationiques à motifs de formule (V) peuvent être préparés selon les procédés décrits dans les brevets US 4 157 388, US 4 390 689, US 4 702 906, US 4 719 282.

Les polymères amphotères sont des polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes;
K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motifs éthylène α,β-dicarboxylique dont l'un des groupements carboxyliques a été amené à réagir avec une polyamine comportant un ou plusieurs groupements amine primaire ou secondaire.
Les polymères amphotères répondant à la définition donnée ci-dessus et satisfaisant la densité de charge cationique ≥ 2 meq/g qui sont plus particulièrement préférés selon l'invention sont choisis dans le groupe formé parles copolymères d'acide acrylique et de diméthyldiallylammonium proposés sous les appellations MERQUAT® 280, MERQUAT® 295 et MERQUAT® PLUS 3330 par la société CALGON.

Les polymères canoniques et amphotères de densité de charge cationique supérieure ou égale à 2 meq par gramme représentent environ de 0,01 à 10% en poids et de préférence d'environ 0,2 à 5% en poids du poids total de la composition de teinture et d'environ 0,0025 à 10% et de préférence d'environ 0,05 à 5% du poids total de celle prête à l'emploi (comprenant l'oxydant).

### Colorants d'oxydation

Les colorants d'oxydation utilisables selon l'invention sont choisis parmi les bases d'oxydation et/ou les coupleurs.
De préférence les compositions selon invention contiennent au moins une base d'oxydation.
Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques suivantes ainsi que leurs sels d'addition avec un acide.
On peut notamment citer :
- (1) les paraphénylènediamines de formule (VII) suivante et leurs sels d'addition avec un acide : 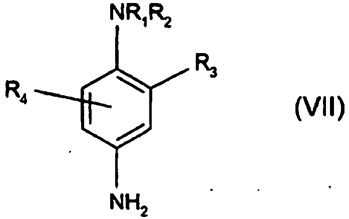 dans laquelle :
   R₁ représente un atome d'hydrogène, un radical alkyle en C₁-C₄, monohydroxyalkyle en C₁-C₄, polyhydroxyalkyle en C₂-C₄. alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréldo;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄.

Parmi les groupements azotés de la formule (VII) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (VII) ci-dessus, on peut plus particulièrement citer la paraphénylènediamine, la paratoluylènediamine, la 2-chloro-paraphénylènediamine, la 2,3-diméthyl-paraphériylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènecoamine, la 4-amino-N,N-diéthyl-3-méthyl-aniline, la N,N-bis-(β-hydroxyéthyl)-paraphénylènediamine, la4-N,N-bis-(β-hydroxyéthyl) amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénytènediamine, la 2-hydroxyméthyl-paraphénylènediamine, la N,N-diméthyl-3-méthyl-paraphénylène-diamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylène-diamine, la 2-méthyl-1-N-β-hydroxyéthylparaphénylènediamine, la N-(4-aminophényl)-3-hydroxy-pyrrolidine, et leurs sels d'addition avec un acide.

Parmi les paraphénylènediamines de formule (VII) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxy-éthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
- (II) Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tirictoriales conformes à l'invention, on peut notamment citer les composés répondant à la formule (VIII) suivante, et leurs sels d'addition avec un acide : 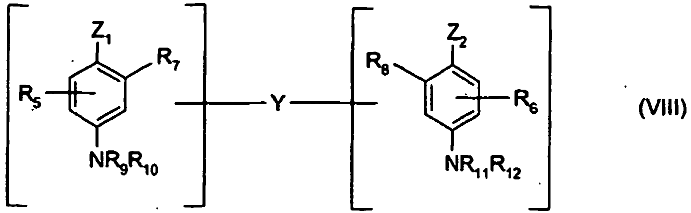 dans laquelle :
- Z, et Z₂, identiques ou différents, représentent un radical hydroxyle ou -NH₂ pouvant être substitué par un radical alkyle en C₁-C₄ ou par un bras de liaison Y;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂ identiques ou différents, représentent un atome d'hydrogène, un bras de liaison Y ou un radical alkyle en C₁-C₄ ;
étant entendu que les composés de formule (VIII) ne comportent qu'un seul bras de liaison Y par molécule.

Parmi les groupements azotés de la formule (VIII) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (VIII) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamlne, la N,N'-bis-(4-méthyl-aminophényl)-tétraméthylènediamine, la N,N'-bis-(éthyl)-N,N'-bis-(4'-amlno-3'-méthylphényl)-éthylènediamine, le 1,8-bis-(2,5-diaminophénoxy)-3,5-dioxaoctane, et leurs sels d'addition avec un acide.

Parmi ces bases doubles de formule (VIII)), le N, N'-bis-β-hydroxyéthyl)-N.N'-bis-(4'-aminophényl)-1,3-diaminopropanol, le 1,8-bis-(2.5-diaminophénoxy)-3,5-dioxaoctane ou l'un de leurs sels d'addition avec un acide sont particulièrement préférés.
- (III) les para-aminophénols répondant à la formule (IX) suivante, et leurs sels d'addition avec un acide : 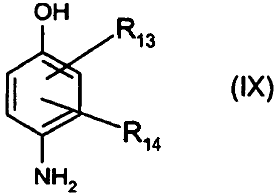 dans laquelle :
   R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, mono-hydroxyalkyle en C₁-C₄, polyhydroxyalkyle en C₂-C₄, aminoalkyle en C₁-C₄, cyanoalkyle en C₁-C₄ ou alcoxy(C₁-C₄) alkyle(C₁-C₄).

Parmi les para-aminophénols de formule (IX) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- (IV) les ortho-aminophénols utilisables à titre de bases d'oxydation dans le cadre de la présente l'Invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
- (V) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine; la 2,5-diméthyl-pyrazolo-[1,5-a]-pyrimidine-3,7-diamine; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazo-10-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrozole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino5-(2'-aminoéthyl)amino-1,3-diméthyl-pyrazole, le 3,4,5-triamino-pynazole, le 1-méthyl-3,4,5-triamino-pyrazole, le 3,5-diamino-1-méthyl-4-méthylamino-pynazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition ne comprenant pas l'oxydant et encore plus préférentiellement de 0,005 à 8% en poids environ de ce poids.

Les coupleurs utilisables dans la composition de teinture selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthytoxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, Pα-naphtol, le 6-hydroxy-indole, le 4-hydroxy-indole, le 4-hydroxy-N-β-hydroxyéthyl-indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Lorsqu'ils sont présents, ces coupleurs représentent de préférence de 0,0001 à 10% en poids environ du poids total de la composition ne comprenant pas l'oxydant, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition tinctoriale conforme à l'invention peut en outre cromprendre un ou plusieurs colorants directs notamment pour modifier les nuances en les enrichissant de reflets. Ces colorants directs peuvent notamment être choisis parmi les colorants nitrés, azoïques ou anthraquinoniques, neutres, cationiques ou anioniques, classiquement utilisés ou ceux décrits notamment dans les demandes de brevet FR-2782450, 2782451, 2782452 et EP-1025834, dans la proportion pondérale d'environ 0,001 à 20% et de préférence de 0,01 à 10% du poids total de la composition.

La composition prête à l'emploi selon l'invention peut également comprendre dans la composition colorante et/ou la composition oxydante des agents d'ajustement de la rhéologie tels que les amides d'acides gras éventuellement oxyéthylénés (diéthanol- ou monoéthanol-amide de coprah, monoéthanolamide d'acide alkyl éther carboxylique oxyéthyléné), les épaississants cellulosiques (hydroxyéthycellulose, hydroxypropyl-cellulose, carboxyméthylcellulose..), la gomme de guar et ses dérivés(hydroxypropylguar..), les gommes d'origine microbienne (gomme de xanthane, gomme de scléroglucane..), les homopolymères réticulés d'acide acrylique ou d'acide acrylamidopropanesulfonique et les polymères associatifs tels que décrits ci-dessous.

### Polymères associatifs utilisables selon l'invention

Les polymères associatifs sont des polymères hydrosolubles capables, dans un milieu aqueux, de s'associer réversiblement entre eux ou avec d'autres molécules.
Leur structure chimique comprend des zones hydrophiles, et des zones hydrophobes caractérisées par au moins une chaîne grasse.
Les polymères associatifs selon l'invention peuvent être de type anionique, cationique, amphotère et de préférence non ionique ou cationique.
Leur concentration en poids dans la composition colorante peut varier d'environ 0,01 à 10% du poids total de la composition et dans la composition prête à l'emploi (comprenant l'oxydant) d'environ 0,0025 à 10% du poids total de la composition. Plus préférentiellement, cette quantité varie d'environ 0,1 à 5% en poids dans la composition colorante et d'environ 0,025 à 10% dans la composition prête à l'emploi.

### Polymères associatifs de type anionique :

On peut citer parmi eux :
- (I) ceux comportant au moins un motif hydrophile, et au moins un motif éther d'allyle à chaîne grasse, plus particulièrement ceux dont le motifs hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (I) suivante :

   CH₂ = C R'CH₂ O Bₙ R (I)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl (C₁₈).
   Des polymères associatifs anioniques de ce type sont décrits et préparés, selon un procédé de polymérisation en émulsion, dans le brevet EP-0 216 479.
   Parmi ces polymères associatifs anioniques, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (I), et de 0 à 1% en poids d'un agent réticulant qui est un monomère insaturé polyéthylénique copolymérisable bien connu, comme le phtalate de diallyle, le (méth) acrylate d'allyl, le divinylbenzène, le diméthacrylate de (poly)éthylèneglycol, et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80® et SALCARE SC90® qui sont des émulsions aqueuses à 30% d'un terpolymère réticulé d'acide méthacrylique, d'acrylate d'éthyle et de steareth-10-allyl éther (40/50/10).
- (II) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (II) suivante : 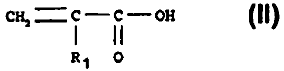 dans laquelle, R, désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (III) suivante 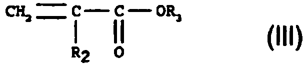 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylate), R₃ désignant un radical alkyle en C₁₀-C₃₀, et de préférence en C₁₂-C₂₂.
Des esters d'alkyles (C₁₀-C₃₀) d'acides carboxyliques insaturés conformes à invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
Des polymères anioniques de ce type sont par exemple décrits et préparés, selon les brevets US-3 915 921 et 4 509 949. Parmi ce type de polymères associatifs anioniques, on utilisera plus particulièrement des polymères formés à partir d'un mélange de monomères comprenant :
(i) essentiellement de l'acide acrylique,
(ii) un ester de formule (III) décrite ci-dessus et dans laquelle R₂ désigne H ou CH₃, R₃ désignant un radical alkyle ayant de 12 à 22 atomes de carbone.
(iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.

Parmi ce type de polymères associatifs anioniques, on utilisera plus particulièrement ceux constitués de 95 à 60% en poids d'acide acrylique (motif hydrophile), 4 à 40% en poids d'acrylate d'alkyles en C₁₀-C₃₀ (motif hydrophobe). et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précédemment.
Parmi lesdits polymères ci-dessus, on préfère tout particulièrement selon la présente invention, les produits vendus par la société GOODRICH sous les dénominations commerciales PEMULENTR1®, PEMULEN TR2®, CARBOPOL 1382®, et encore plus préférentiellement le PEMULENTR1®, et le produit vendu par la société S.E.P.P.I.C. sous la dénomination COATEX SX®.
- (III) les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608® par la société NEWPHASE TECHNOLOGIES.
- (IV) les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique nontensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (40OE) en dispersion aqueuse à 25%.
- (V) les copolymères comportant parmi leurs monomères un acide carboxylique à insaturation α,β-monoéthylénique et un ester d'acide carboxylique à insaturation α,β-monoéthylénique et d'un alcool gras oxyalkyléné. Préférentiellement ces composés comprennent également comme monomère un ester d'acide carboxylique à insaturation α,β-monoéthylénique et d'alcool en C1C4.
   A titre d'exemple de ce type de composé on peut citer l'ACULYN 22® vendu par la société ROHM et HAAS, qui est un terpolymère acide méthacrylique/acrylate d'éthyle/méthacrylate de stéaryle oxyalkyléné.

### Polymères associatifs de type cationique

On peut citer parmi eux :
- (I) les polyuréthanes associatifs cationiques dont la famille a été décrite par la demanderesse dans la demande de brevet français N°-0009609; elle peut être représentée par la formule générale (la) suivante :

   R-X-(P)ₙ-[L-(Y)ₘ]ᵣ-L'-(P')ₚ-X'-R' (la)

   dans laquelle :
   R et R', identiques ou différents, représentent un groupement hydrophobe ou un atome d'hydrogène ;
   X et X', identiques ou différents, représentent un groupement comportant une fonction amine portant ou non un groupement hydrophobe, ou encore le groupement L" ;
   L, L'et L", identiques ou différents, représentent un groupement dérivé d'un diisocyanate ;
   P et P', identiques ou différents, représentent un groupement comportant une fonction amine portant ou non un groupement hydrophobe ;
   Y représente un groupement hydrophile ;
   r est un nombre entier compris entre 1 et 100, de préférence entre 1 et 50 et en particulier entre 1 et 25,
   n, m, et p valent chacun indépendamment des autres entre 0 et 1000 ; la molécule contenant au moins une fonction amine protonée ou quatemisée et au moins un groupement hydrophobe.

Dans un mode de réalisation préféré de ces s polyuréthanes, les seuls groupements hydrophobes sont les groupes R et R' aux extrémités de chaîne.

Une famille préférée de polyuréthanes associatifs cationiques est celle correspondant à la formule (la) décrite ci-dessus et dans laquelle :
R et R' représentent tous les deux indépendamment un groupement hydrophobe,
X, X' représentent chacun un groupe L",
n et p valent entre 1 et 1000 et
L, L', L", P, P', Y et m ont la signification indiquée ci-dessus.

Une autre famille préférée de polyuréthanes associatifs cationiques est celle correspondant à la formule (la) ci-dessus dans laquelle :
R et R' représentent tous les deux indépendamment un groupement hydrophobe, X,
X' représentent chacun un groupe L", n et p valent 0, et L, L', L", Y et m ont la signification indiquée ci-dessus.

Le fait que n et p valent 0 signifie que ces polymères ne comportent pas de motifs dérivés d'un monomère à fonction amine, incorporé dans le polymère lors de la polycondensation. Les fonctions amine protonées de ces polyuréthanes résultent de l'hydrolyse de fonctions isocyanate, en excès, en bout de chaîne, suivie de l'alkylation des fonctions amine primaire formées par des agents d'alkylation à groupe hydrophobe, c'est-à-dire des composés de type RQ ou R'Q, dans lequel R et R' sont tels que définis plus haut et Q désigne un groupe partant tel qu'un halogénure, un sulfate etc.

Encore une autre famille préférée de polyuréthanes associatifs cationiques est celle correspondant à la formule (la) ci-dessus dans laquelle :
R et R' représentent tous les deux indépendamment un groupement hydrophobe,
X et X' représentent tous les deux indépendamment un groupement comportant une amine quaternaire,
n et p valent zéro, et
L, L', Y et m ont la signification indiquée ci-dessus.

La masse moléculaire moyenne en nombre des polyuréthanes associatifs cationiques est comprise de préférence entre 400 et 500 000, en particulier entre 1000 et 400 000 et idéalement entre 1000 et 300 000.
Par groupement hydrophobe, on entend un radical ou polymère à chaîne hydrocarbonée, saturée ou non, linéaire ou ramifiée, pouvant contenir un ou plusieurs hétéroatomes tels que P, O, N, S, ou un radical à chaîne perfluorée ou siliconée. Lorsqu'il désigne un radical hydrocarboné, le groupement hydrophobe comporte au moins 10 atomes de carbone, de préférence de 10 à 30 atomes de carbone, en particulier de 12 à 30 atomes de carbone et plus préférentiellement de 18 à 30 atomes de carbone.
Préférentiellement, le groupement hydrocarboné provient d'un composé monofonctionnel.
A titre d'exemple, le groupement hydrophobe peut être issu d'un alcool gras tel que l'alcool stéarylique, l'alcool dodécylique, l'alcool décylique. Il peut également désigner un polymère hydrocarboné tel que par exemple le polybutadiène.

Lorsque X et/ou X' désignent un groupement comportant une amine tertiaire ou quaternaire, X et/ou X' peuvent représenter l'une des formules suivantes : 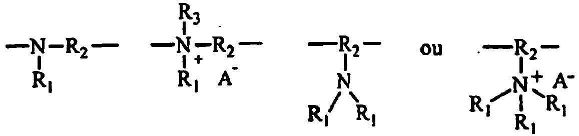 pour X 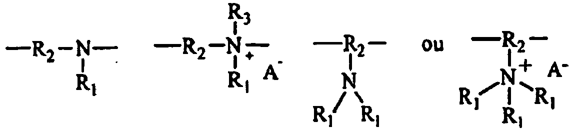 pour X' dans lesquelles :
R₂ représente un radical alkylène ayant de 1 à 20 atomes de carbone, linéaire ou ramifié, comportant ou non un cycle saturé ou insaturé, ou un radical arylène, un ou plusieurs des atomes de carbone pouvant être remplacé par un hétéroatome choisi parmi N, S, O, P ;
R₁ et R₃, identiques ou différents, désignent un radical alkyle ou alcényle en C₁-C₃₀, linéaire ou ramifié, un radical aryle, l'un au moins des atomes de carbone pouvant être remplacé par un hétéroatome choisi parmi N, S, O, P ;
A⁻ est un contre-ion physiologiquement acceptable.

Les groupements L, L' et L" représentent un groupe de formule : 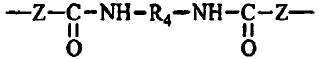 dans laquelle :
Z représente -O-, -S- ou -NH- ; et
R₄ représente un radical alkylène ayant de 1 à 20 atomes de carbone, linéaire ou ramifié, comportant ou non un cycle saturé ou insaturé, un radical arylène, un ou plusieurs des atomes de carbone pouvant être remplacé par un hétéroatome choisi parmi N, S, O et P.

Les groupements P et P', comprenant une fonction amine peuvent représenter au moins l'une des formules suivantes : 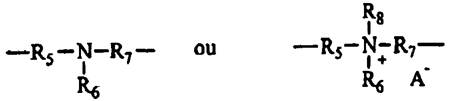 ou 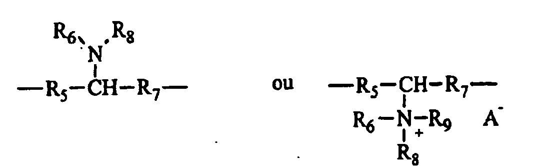 ou 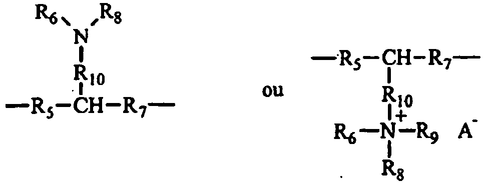 dans lesquelles :
R₅ et R₇ ont les mêmes significations que R₂ défini précédemment;
R₆, R₈ et R₉ ont les mêmes significations que R₁ et R₃ définis précédemment ;
R₁₀ représente un groupe alkylène, linéaire ou ramifié, éventuellement insaturé et pouvant contenir un ou plusieurs hétéroatomes choisis parmi N, O, S et P,
et A- est un contre-ion physiologiquement acceptable.

En ce qui concerne la signification de Y, on entend par groupement hydrophile, un groupement hydrosoluble polymérique ou non.
A titre d'exemple, on peut citer, lorsqu'il ne s'agit pas de polymères, l'éthylèneglycol, le diéthylèneglycol et le propylèneglycol.
Lorsqu'il s'agit, conformément à un mode de réalisation préféré, d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Les polyuréthanes associatifs cationiques de formule (Ia) selon l'invention sont formés à partir de diisocyanates et de différents composés possédant des fonctions à hydrogène labile. Les fonctions à hydrogène labile peuvent être des fonctions alcool, amine primaire ou secondaire ou thiol donnant, après réaction avec les fonctions diisocyanate, respectivement des polyuréthanes, des polyurées et des polythiourées. Le terme "polyuréthanes" de la présente invention englobe ces trois types de polymères à savoir les polyuréthanes proprement dits, les polyurées et les polythiourées ainsi que des copolymères de ceux-ci.

Un premier type de composés entrant dans la préparation du polyuréthane de formule (la) est un composé comportant au moins un motif à fonction amine. Ce composé peut être multifonctionnel, mais préférentiellement le composé est difonctionnel, c'est-à-dire que selon un mode de réalisation préférentiel, ce composé comporte deux atomes d'hydrogène labile portés par exemple par une fonction hydroxyle, amine primaire, amine secondaire ou thlol. On peut également utiliser un mélange de composés multifonctionnels et difonctionnels dans lequel le pourcentage de composés multifonctionnels est faible.
Comme indiqué précédemment, ce composé peut comporter plus d'un motif à fonction amine. Il s'agit alors d'un polymère portant une répétition du motif à fonction amine. Ce type de composés peut être représenté par l'une des formules suivantes :

HZ-(P)ₙ-ZH,

ou

HZ-(P')ₚ-ZH

dans lesquelles Z, P, P', n et p sont tels que définis plus haut.

A titre d'exemple de composé à fonction amine, on peut citer la N-méthyldiéthanolamine, la N-tert-butyl-diéthanolamine, la N-sulfoéthyldiéthanolamine.

Le deuxième composé entrant dans la préparation du polyuréthane de formule (la) est un diisocyanate correspondant à la formule :

O=C=N-R₄-N=C=O

dans laquelle R₄ est défini plus haut.
A titre d'exemple, on peut citer le méthylènediphényl-diisocyanate, le méthylènecyclohexanediisocyanate, l'isophoronediisocyanate, le toluène-diisocyanate, le naphtalènediisocyanate, le butanediisocyanate, l'hexane-diisocyanate.

Un troisième composé entrant dans la préparation du polyuréthane de formule (la) est un composé hydrophobe destiné à former les groupes hydrophobes terminaux du polymère de formule (la).
Ce composé est constitué d'un groupe hydrophobe et d'une fonction à hydrogène labile, par exemple une fonction hydroxyle, amine primaire ou secondaire, ou thiol.
A titre d'exemple, ce composé peut être un alcool gras, tel que notamment l'alcool stéarylique, l'alcool dodécylique, l'alcool décylique. Lorsque ce composé comporte une chaîne polymérique, il peut s'agir par exemple du polybutadiène hydrogéné □-hydroxyle.

Le groupe hydrophobe du polyuréthane de formule (la) peut également résulter de la réaction de quaternisation de l'amine tertiaire du composé comportant au moins un motif amine tertiaire. Ainsi, le groupement hydrophobe est introduit par l'agent quatemisant Cet agent quaternisant est un composé de type RQ ou R'Q, dans lequel R et R' sont tels que définis plus haut et Q désigne un groupe partant tel qu'un halogénure, un sulfate etc.

Le polyuréthane associatif cationique peut en outre comprendre une séquence hydrophile. Cette séquence est apportée par un quatrième type de composé entrant dans la préparation du polymère. Ce composé peut être multifonctionnel. Il est de préférence difonctionnel. On peut également avoir un mélange où le pourcentage en composé multifonctionnel est faible.

Les fonctions à hydrogène labile sont des fonctions alcool, amine primaire ou secondaire, ou thiol. Ce composé peut être un polymère terminé aux extrémités des chaînes par l'une de ces fonctions à hydrogène labile.
A titre d'exemple, on peut citer, lorsqu'il ne s'agit pas de polymères, l'éthylèneglycol, le diéthylèneglycol et le propylèneglycol.
Lorsqu'il s'agit d'un polymère hydrophile, on peut citer à titre d'exemple les polyéthers, les polyesters sulfonés, les polyamides sulfonés, ou un mélange de ces polymères. A titre préférentiel, le composé hydrophile est un polyéther et notamment un poly(oxyde d'éthylène) ou poly(oxyde de propylène).

Le groupe hydrophile noté Y dans la formule (Ia) est facultatif. En effet, les motifs à fonction amine quaternaire ou protonée peuvent suffire à apporter la solubilité ou l'hydrodispersibilité nécessaire pour ce type de polymère dans une solution aqueuse. Bien que la présence d'un groupe Y hydrophile soit facultative, on préfère cependant des polyuréthanes associatifs cationiques comportant un tel groupe.
- (II) les dérivés de cellulose quatemisée et les polyacrylates à groupements latéraux aminés non cycliques.

Les dérivés de cellulose quatemisée sont en particulier,
- les celluloses quatemisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.
Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.

On peut indiquer comme exemples d'alkylhydroxyéthyl-celluloses quatemisées à chaînes grasses en C₈-C₃₀, les produits QUATRISOFT LM 200®, QUATRISOFT LM-X 529-18-A®, QUATRISOFT LM-X 529-18B® (alkyle en C₁₂) et QUATRISOFT LM-X 529-8® (alkyle en C₁₈) commercialisés par la société AMERCHOL et les produits CRODACEL QM®, CRODACEL QL® (alkyle en C₁₂) et CRODACEL QS® (alkyle en C18) commercialisés par la société CRODA.

### Polymères associatifs amphotères

Ils sont choisis de préférence parmi ceux comportant au moins un motif cationique non cyclique. Plus particulièrement encore, on préfère ceux préparés à partir ou comprenant 1 à 20 moles% de monomère comportant une chaîne grasse, et de préférence 1,5 à 15 moles% et plus particulièrement encore 1,5 à 6 moles%, par rapport au nombre total de moles de monomères.

Les polymères associatifs amphotères préférés selon invention comprennent, ou sont préparés en copolymérisant :
1) au moins un monomère de formule (la) ou (Ib): 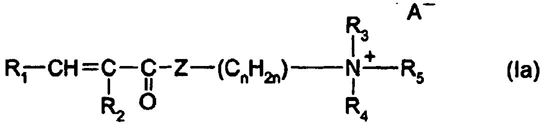 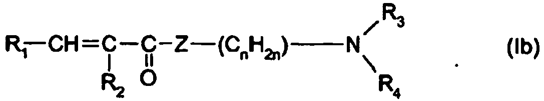
   dans lesquelles, R₁ et R₂, identiques ou différents, représentent un atome d'hydrogène ou un radical méthyle, R₃, R₄ et R₅, identiques ou différents, représente un radical alkyle linéaire ou ramifié ayant de 1 à 30 atomes de carbone,
   Z représente un groupe NH ou un atome d'oxygène,
   n est un nombre entier de 2 à 5,
   A- est un anion issu d'un acide organique ou minéral, tel qu'un anion méthosulfate ou un halogénure tel que chlorure ou bromure;
2) au moins un monomère de formule (II)

   R₆-CH=CR₇-COOH (II)

   dans laquelle, R₆ et R₇, identiques ou différents, représentent un atome d'hydrogène ou un radical méthyle; et
3) au moins un monomère de formule (III):

   R⁶-CH=CR₇-COXR₈ (III)

   dans laquelle R₆ et R₇, identiques ou différents, représentent un atome d'hydrogène ou un radical méthyle, X désigne un atome d'oxygène ou d'azote et R₈ désigne un radical alkyle linéaire ou ramifié ayant de 1 à 30 atomes de carbone ; l'un au moins des monomères de formule (la), (Ib) ou (III) comportant au moins une chaîne grasse.

Les monomères de formule (la) et (Ib) de la présente invention sont choisis, de préférence, dans le groupe constitué par :
- le diméthylaminoéthylméthacrylate, le diméthylaminoéthylacrylate,
- le diéthylaminoéthylméthacrylate, le diéthylaminoéthylacrylate,
- le diméthylaminopropylméthacrylate, le diméthylaminopropylacrylate,
- le diméthylaminopropylméthacrylamide, le diméthylaminopropylacrylamide, ces monomères étant éventuellement quaternisés, par exemple par un halogénure d'alkyle en C₁-C₄ ou un sulfate de dialkyle en C₁-C₄.

Plus particulièrement, le monomère de formule (Ia) est choisi parmi le chlorure d'acrylamidopropyl triméthyl ammonium et le chlorure de méthacrylamidopropyl triméthyl ammonium.

Les monomères de formule (II) de la présente invention sont choisis, de préférence, dans le groupe constitué par l'acide acrylique, l'acide méthacrylique, l'acide crotonique et l'acide méthyl-2 crotonique. Plus particulièrement, le monomère de formule (II) est l'acide acrylique.

Les monomères de formule (III) de la présente invention sont choisis, de préférence, dans le groupe constitué par des acrylates ou méthacrylates d'alkyle en C₁₂-C₂₂ et plus particulièrement en C₁₆-C₁₈.

Les monomères constituant les polymères amphotères à chaîne grasse de l'invention sont de préférence déjà neutralisés et/ou quaternisés.

Le rapport du nombre de charges cationiques/charges anioniques est de préférence égal à environ 1.

Les polymères associatifs amphotères selon l'invention comprennent de préférence de 1 à 10 moles% du monomère comportant une chaîne grasse (monomère de formule (la), (Ib) ou (III)), et de préférence de 1,5 à 6 moles %.

Les poids moléculaires moyens en poids des polymères associatifs amphotères selon l'invention peuvent varier de 500 à 50.000.000 et sont de préférence compris entre 10.000 et 5 000 000.

Les polymères associatifs amphotères selon l'invention peuvent également contenir d'autre monomères tels que des monomères non ioniques et en particulier tels que les acrylates ou méthacrylates d'alkyle en C₁-C₄.

Des polymères associatifs amphotères selon l'invention sont par exemple décrits et préparés dans la demande de brevet WO9844012 Parmi les polymères associatifs amphotères selon l'invention, on préfère les terpolymères acide acrylique/chlorure de (méth)acrylamidopropyl triméthyl ammonium/ méthacrylate de stéaryle.

### Polymères associatifs de type non ionique

Selon l'invention, ils sont choisis de préférence parmi :
- (1) les celluloses modifiées par des groupements comportant au moins une chaîne grasse ; on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS® (alkyles en C₁₆) vendu par la société AQUALON, ou le produit BERMOCOLL EHM 100® vendu par la société BEROL NOBEL,
   - celles modifiées par des groupes polyalkylène glycol éther d'alkyl phénol, tel que le produit AMERCELL POLYMER HM-1500® (polyéthylène glycol (15) éther de nonyl phénol) vendu par la société AMERCHOL.
- (2) les hydroxypropylguars modifiés par des groupements comportant au moins une chaîne grasse tel que le produit ESAFLOR HM 22® (chaîne alkyle en C₂₂) vendu par la société LAMBERTI, les produits RE210-18® (chaîne alkyle en C₁₄) et RE205-1® (chaîne alkyle en C₂₀) vendus par la société RHONE POULENC.
- (3) les copolymères de vinyl pyrrolidone et de monomères hydrophobes à chaîne grasse dont on peut citer à titre d'exemple :
   - les produits ANTARON V216® ou GANEX V216® (copolymère vinylpyrrolidone /hexadécène) vendu par la société I.S.P.
   - les produits ANTARON V220® ou GANEX V220® (copolymère vinyIpyrrolidone/eicosène) vendu par la société I.S.P.
   - (4) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208®.
   - (5) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
   - (6) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
   - (7) les polymères à squelette aminoplaste éther possédant au moins une chaîne grasse, tels que les composés PURE THIX® proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom. Par extension figurent aussi parmi les polyéthers polyuréthanes non-ioniques à chaîne grasse, ceux dont les séquences hydrophiles sont liées aux séquences lipophiles par d'autres liaisons chimiques.
A titre d'exemples de polyéthers polyuréthanes non-ioniques à chaîne grasse utilisables dans l'invention, on peut aussi utiliser aussi le Rhéolate 205® à fonction urée vendu par la société RHEOX ou encore les Rhéolates® 208 , 204 ou 212, ainsi que l'Acrysol RM 184®.

On peut également citer le produit ELFACOS T210® à chaîne alkyle en C₁₂₋₁₄ et le produit ELFACOS T212® à chaîne alkyle en C₁₈ de chez AKZO.
Le produit DW 1206B® de chez ROHM & HAAS à chaîne alkyle en C₂₀ et à liaison uréthanne, proposé à 20 % en matière sèche dans l'eau, peut aussi être utilisé.
On peut aussi utiliser des solutions ou dispersions de ces polymères notamment dans l'eau ou en milieu hydroalcoolique. A titre d'exemple, de tels polymères on peut citer, le Rhéolate® 255, le Rhéolate® 278 et le Rhéolate® 244 vendus par la société RHEOX. On peut aussi utiliser le produit DW 1206F et le DW 1208J proposés par la société ROHM & HAAS. Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Plus particulièrement encore, selon l'invention, on préfère utiliser un polyéther polyuréthane susceptible d'être obtenu par polycondensation d'au moins trois composés comprenant (i) au moins un polyéthylèneglycol comprenant de 150 à 180 moles d'oxyde d'éthylène, (ii) de l'alcool stéarylique ou de l'alcool décylique et (iii) au moins un diisocyanate. De tels polyéther polyuréthanes sont vendus notamment par la société ROHM & HAAS sous les appellations Aculyn 46® et Aculyn 44® [l'ACULYN 46® est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44® est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

### Milieu

Le milieu de la composition colorante approprié pour la teinture, est de préférence un milieu aqueux constitué par de l'eau pouvant comprendre avantageusement des solvants organiques acceptables sur le plan cosmétique, dont plus particulièrement, des alcools ou diols, tels que l'alcool éthylique, l'alcool isopropylique, l'hexylèneglycol (2-méthyl 2,4-pentanediol), le néopentylglycol et le 3-méthyl-1,5-pentanediol, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme parexemple, le monoéthyléther ou le monobutyléther du diéthylèneglycol. Les solvants peuvent alors être présents, chacun, dans des concentrations comprises entre environ 0,5 et 20% et, de préférence, entre environ 2 et 10% en poids par rapport au poids total de la composition.

### Autres adjuvants

La composition colorante selon l'invention peut encore comprendre une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiant, etc....

Ladite composition peut également comprendre des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids totale de la composition.

La composition colorante selon l'invention peut également comprendre de préférence au moins un alcool gras additionnel, ces alcools gras étant introduits sous forme pure ou de mélange. On peut citer parmi eux plus particulièrement les alcools laurique, cétylique, stéarylique, oléique et leurs mélanges. Ces alcools gras additionnels peuvent représenter de 0,001 à 20% en poids environ du poids total de la composition.

Bien entendu, l'homme de l'art veillera à choisir le ou les éventuels composés complémentaires mentionnés ci-avant, de manière telle que les propriétés avantageuses attachées intrinsèquement à la composition prête à l'emploi selon l'invention ne soient pas, ou substantiellement pas, altérées par la ou les adjonctions envisagées.

### Oxydant

Dans la composition oxydante, l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition prête à l'emploi et appliquée sur les fibres kératiniques [composition résultant du mélange de la composition tinctoriale et de la composition oxydante], est généralement compris entre les valeurs 3 et 12, bornes incluses. Il est de préférence compris entre 8,5 et 11 bornes Incluses, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule suivante : 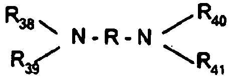 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition prête à l'emploi, réalisée extemporanément au moment de l'emploi à partir des compositions colorante et oxydante décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Un exemple concret illustrant invention est indiqué ci-après, sans pour autant présenter un caractère limitatif.

### EXEMPLE 1

On a préparé la composition de teinture suivante :

**Composition colorante : (exprimée en grammes de Matière active)**

| | |
|---|---|
| Acide lauryl éther carboxylique 4,5 OE (Akypo® RLM 45 vendu par CHEM Y) | 7 |
| Alcool laurique 2 OE (Dehydol®LS-2-DEO-N vendu par COGNIS) | 4 |
| Alcool décylique 5OE (Empilan®KA-5/90-FL vendu par ALBRIGHT & WILSON) | 8 |
| Alcool oléique | 3 |
| Monoéthanolamide d'acide alky (C13/C15) éther carboxylique à 2 moles d'oxyde d'éthylène | 5 |
| Polymère associatif non ionique (Dapral® T212 vendu par AKZO) | 1 |
| Monoéthanolamine | 2 |
| Polyquaternium 6 (Merquat® 100 vendu par CALGON) | 1,5 |
| Ethanol | 11 |
| Propylène glycol | 5 |
| Dipropylène glycol | 5 |
| 1,3-dihydroxybenzène (résorcinol) | 0,3 |
| Paraphénylènediamine | 0,3 |
| Réducteurs, antioxydants | q.s. |
| Séquestrant | q.s. |
| Parfum | q.s. |
| Ammoniaque (à 20,5% en ammoniac) | 1,6 |
| Eau déminéralisée qsp | 100 |

La composition colorante a été mélangée, au moment de remploi, dans un bol en plastique et pendant 2 minutes, à une composition oxydante titrant 20 volumes en eau oxygénée à raison de 1 partie de composition colorante pour 1,5 partie de composition oxydante.
On a appliqué le mélange obtenu sur des mèches de cheveux à 90% de blancs et on a laissé poser 30 minutes.
On a ensuite rincé les mèches à l'eau, on les a lavées au shampooing, à nouveau rincées à l'eau, puis séchées et démêlées.
On a obtenu alors une nuance brun verdâtre.

### EXEMPLE 2

On a préparé la composition de teinture suivante :

**Composition colorante : (exprimée en grammes de Matière active)**

| | |
|---|---|
| Alcoyl C12 éther de glycérol (1,5 moles) | 4 |
| hexylène glycol (2 methyl-2,4 pentanediol) | 5 |
| Alcool éthylique 96 degrés dénaturé | 9 |
| Acide lauryl éther carboxylique (4.5 OE) | 8 |
| Amide d'acides de colza oxyéthyléné (4 OE) | 6 |
| Alcool oléique | 2 |
| Lauryl hydroxyethylcellulose quaternisée | 1 |
| Alcool décylique oxyéthyléné (5 OE) | 6 |
| Thiolactate d'ammonium en solution aqueuse à 58 % (50 % en acide thiolactique) | 0.8 |
| Polycondensat tétraméthyl hexaméthylènediamine / dichloro 1,3-propylène en solution aqueuse | 2 |
| 3-méthyl-1-phényl-5-pyrazolone | 0.15 |
| 1,3-dihydroxybenzène (résorcinol) | 0.056 |
| 1-hydroxy-3-amino-benzène | 0.31 |
| 1,4-diamino-benzène | 0.4 |
| Acide éthylène diamine tétracétique | 0.2 |
| Monoéthanolamine pure | 1.1 |
| Acide érythorbique (ou acide d-isoascorbique) | 0.12 |
| Parfum | 0.7 |
| Ammoniaque (concentration de référence à 20%) | 8 |
| Dipropylène glycol | 6 |
| Eau désionisée | Qsp 100 |

La composition colorante a été mélangée, au moment de l'emploi, dans un bol en plastique et pendant 2 minutes, à une composition oxydante titrant 20 volumes en eau oxygénée à raison de 1 partie de composition colorante pour 1,5 partie de composition oxydante.
On a appliqué le mélange obtenu sur des mèches de cheveux à 90% de blancs et on a laissé poser 30 minutes.
On a ensuite rincé les mèches à l'eau, on les a lavées au shampooing, à nouveau rincées à l'eau, puis séchées et démêlées.
On a obtenu alors une nuance brun verdâtre.

## Revendications

1. Composition pour la teinture d'oxydation pour fibres kératiniques, en particulier pour fibres kératiniques humaines et plus particulièrement des cheveux, comprenant dans un milieu approprié pour la teinture, au moins un colorant d'oxydation, **caractérisée par le fait qu'**elle comprend en outre au moins un acide éther carboxylique polyoxyalkyléné ou l'un de ses sels, au moins un agent tensioactif non-ionique et au moins un polymère cationique ou amphotère dont la densité de charge cationique est supérieure ou égale à 2 meq/gramme, dans laquelle
- les acides éther carboxyliques polyoxyalkylénés ou leurs sels sont de formule (I) suivante : 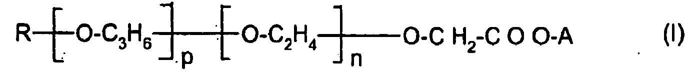 dans laquelle :
R représente un radical ou un mélange de radicaux alkyle ou alcényle linéaire ou ramifié en C₈-C₂₂,
n est un nombre entier ou décimal allant de 2 à 24,
p est un nombre entier ou décimal allant de 0 à 6,
A désigne un atome d'hydrogène ou bien Na, K, Li, 1/2Mg ou un reste monoéthanolamine, ammonium ou triéthanolamine,
- les tensioactifs non ioniques sont choisis dans le groupe formé par :
1) les alcools gras comportant de 8 à 22 atomes de carbone et oxyéthylénés par 1 à 10 moles d'oxyde d'éthylène.
2) les alcools gras mono- ou poly-glycérolés de formule (II) suivante : 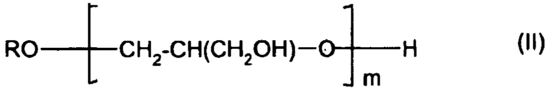 dans laquelle :
R représente un radical saturé ou insaturé, linéaire ou ramifié, comportant de 8 à 40 atomes de carbone et de préférence de 10 à 30 atomes de carbone;
m représente un nombre allant de 1 à 30 et de préférence de 1 à 10.

2. Composition selon la revendication 1, **caractérisée par le fait que** dans la formule (I), R désigne un radical alkyl(C₁₂), A désigne un atome d'hydrogène ou de sodium, p=0, et n varie de 2 à 10.

3. Composition selon la revendication 1, **caractérisée par** te fait que les alcools gras mono- ou poly-glycérolés de formule (II) sont choisis parmi l'alcool en C8/C10 à une mole de glycérol, l'alcool en C10/C12 à 1 mole de glycérol et l'alcool en C12 à 1,5 mole de glycérol.

4. Composition selon l'une quelconque des revendications précédentes, **caractérisée par le fait que** les polymères cationiques ou amphotères de densité de charge cationique supérieure ou égale à 2 meq/ gramme, sont choisis dans le groupe formé par :
1)- les polymères quaternaires de vinylpyrrolidone et de vinylimidazole
2)- les homopolymères de chlorure de diméthyldiallylammonium
3- les copolymères de chlorure de diméthyldiallylammonium et d'acrylamide
4)- les copolymères d'acide acrylique et de diméthyldiallylammonium
5- les polymères de diammonium quaternaire constitués de motifs de formule (V) suivante: 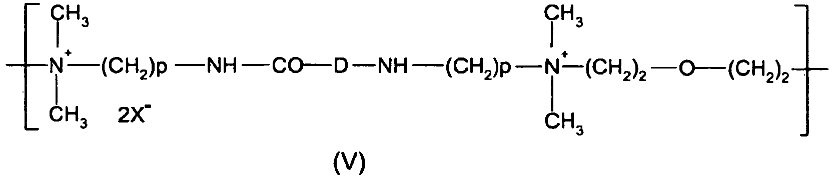 dans laquelle :
p désigne un nombre entier variant de 1 à 6 environ,
D peut être nul ou peut représenter un groupement -(CH₂)ᵣ-CO- dans lequel r désigne un nombre égal à 4 ou à 7, et
X⁻ est un anion.
6- les polymères de diammonium quaternaire contenant des motifs récurrents de formule (IV)bis suivante : 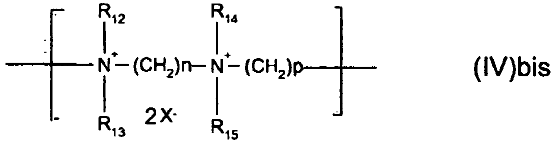 dans laquelle R₁₂, R₁₃, R₁₄ et R₁₅, identiques ou différents, désignent un radical alkyle ou hydroxyalkyle ayant de 1 à 4 atomes de carbone environ, n et p sont des nombres entiers variant de 2 à 20.

5. Composition selon la revendication 4, **caractérisée par le fait que** dans la formule (V) des polymères alinéa (5), p est égal à 3, et,
a) D représente un groupement -(CH₂)₄-CO-, X désigne un atome de chlore,
b) D représente un groupement -(CH₂)₇-CO-, X désigne un atome de chlore,
c) D désigne la valeur zéro, X désigne un atome de chlore,
d) un " Block Copolymer " formé de motifs correspondant aux polymères décrits aux alinéas a) et c).

6. Composition selon la revendication 4, **caractérisée par le fait que** dans la formule (IV)bis, alinéa 6, R₁₂, R₁₃, R₁₄ et R₁₅ représentent un radical méthyle et n = 3, p = 6 et X = Cl,
ou R₁₂ et R₁₃ représentent un radical méthyle, R₁₄ et R₁₅ représentent un radical éthyle et n = p = 3 et X = Br.

7. Composition selon l'une quelconque des revendications précédentes, **caractérisée par le fait que** les acides éther carboxyliques oxyalkylénés ou leurs sels représentent en poids à 15%, et de préférence 3 à 10% du poids total de la composition.

8. Composition selon l'une quelconque des revendications précédentes, **caractérisée par le fait que** les tensioactifs non-ioniques représentent en poids 2 à 40%, et de préférence 4 à 20% du poids total de la composition.

9. Composition selon l'une quelconque des revendications précédentes, **caractérisée par le fait que** les polymères cationiques ou amphotères de densité de charge cationique ≥ 2 meq/gramme représentent en poids 0,01% à 10%, et de préférence 0,2% à 5% du poids total de la composition.

10. Composition selon l'une quelconque des revendications précédentes, **caractérisée par le fait que** le colorant d'oxydation est choisi parmi les bases d'oxydation et les coupleurs.

11. Composition selon la revendication 10, **caractérisée par le fait qu'**elle comprend au moins une base d'oxydation.

12. Composition selon les revendications 10 ou 11, **caractérisée par le fait que** les bases d'oxydation sont choisies parmi les ortho- ou para- phénylènediamines, les bases doubles, les ortho- ou para- aminophénols, et les bases hétérocycliques, ainsi que les sels d'addition de ces composés avec un acide.

13. Composition selon la revendication 12, **caractérisée par le fait que** les para-phénylènediamines sont choisies parmi les composés de formule (VII) suivante : 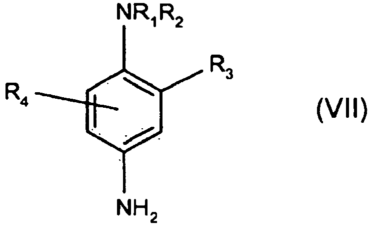 dans laquelle :
R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle;
R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.

14. Composition selon la revendication 12, **caractérisée par le fait que** les bases doubles sont choisies parmi les composés de structure (VIII) suivante : 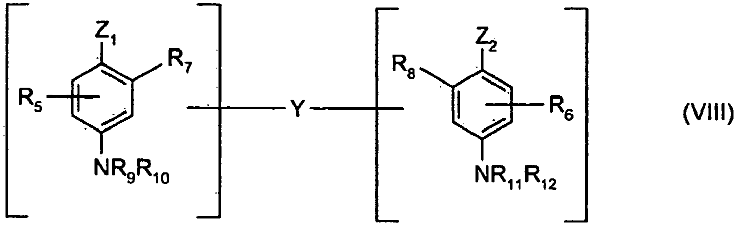 dans laquelle:
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄;
étant entendu que les composés de formule (VIII) ne comportent qu'un seul bras de liaison Y par molécule.

15. Composition selon les revendications 13 ou 14, **caractérisée par le fait que** les groupements azotés sont choisis parmi les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

16. Composition selon la revendication 12, **caractérisée par le fait que** les para-aminophénols sont choisis parmi les composés de structure (IX) suivante : 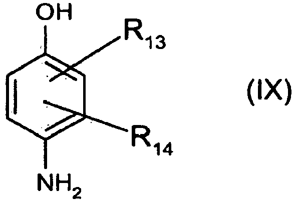 dans laquelle :
R₁₃ représente un atome d'hydrogène, un atome d'halogène, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
R₁₄ représente un atome d'hydrogène ou un atome d'halogène, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

17. Composition selon la revendication 12, **caractérisée par le fait que** les bases hétérocycliques sont choisies parmi les dérivés pyridiniques, les dérivés pyrimidiniques dont les pyrazolopyrimidines, et les dérivés pyrazoliques.

18. Composition selon les revendications 10 à 17, **caractérisée par le fait que** les bases d'oxydation sont présentes dans des concentrations allant de 0,0005 à 12% et de préférence de 0,005 à 8% en poids par rapport au poids total de la composition.

19. Composition selon la revendication 10, **caractérisée par le fait que** les coupleurs sont choisis parmi les métaphénylènediamines, les métaaminophénols, les métadiphénols, les coupleurs hétérocycliques, et les sels d'addition de ces composés avec un acide.

20. Composition selon les revendications 10 ou 19, **caractérisée par le fait que** les coupleurs sont présents dans des concentrations allant de 0,0001 à 10% et de préférence de 0,005 à 5% en poids par rapport au poids total de la composition.

21. Composition selon les revendications 12 ou 19, **caractérisée par le fait que** les sels d'addition avec un acide des bases d'oxydation et des coupleurs sont choisis parmi les chlorhydrates, les bromhydrates, les sulfates, les tartrates, les lactates et les acétates.

22. Composition selon l'une quelconque des revendications précédentes, **caractérisée par le fait qu'**elle comprend en outre des colorants directs.

23. Composition selon l'une quelconque des revendications précédentes, **caractérisée par le fait qu'**elle comprend en outre au moins un agent réducteur, dans des quantités allant de 0,05 à 3% en poids par rapport au poids total de la composition.

24. Composition selon l'une quelconque des revendications précédentes, **caractérisée par le fait qu'**elle comprend au moins un alcool gras additionnel dans une proportion en poids allant de 0,001 à 20% du poids total de la composition.

25. Composition selon l'une quelconque des revendications précédentes, **caractérisée par le fait qu'**elle comprend au moins un polymère associatif dans une proportion allant de 0,01 à 10% du poids total de la composition.

26. Composition prête à l'emploi pour la teinture d'oxydation des fibres kératiniques en particulier des fibres kératiniques humaines telles que les cheveux, **caractérisée par le fait qu'**elle est obtenue par mélange d'une composition colorante telle que définie à l'une quelconque des revendications 1 à 25 et d'une composition oxydante comprenant au moins un agent oxydant.

27. Composition selon la revendication 26, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, le cas échéant en présence de leur donneur ou cofacteur respectif.

28. Composition selon la revendication 27, **caractérisée par le fait que** l'agent oxydant est le peroxyde d'hydrogène.

29. Composition selon la revendication 28, **caractérisée par le fait que** l'agent oxydant est une solution d'eau oxygénée dont le titre varie de 1 à 40 volumes.

30. Composition selon l'une quelconque des revendications précédentes, **caractérisée par le fait qu'**elle possède un pH allant de 3 à 12.

31. Composition selon l'une quelconque des revendications 26 à 30, **caractérisée par le fait que** les acides éther carboxyliques oxyalkylénés ou leurs sels représentent en poids 0,5 à 15%, et de préférence 0,7 à 10% du poids total de la composition.

32. Composition selon l'une quelconque des revendications 26 à 30, **caractérisée par le fait que** les tensioactifs non-ioniques représentent en poids 0,5 à 40%, et de préférence 1 à 20% du poids total de la composition.

33. Composition selon l'une quelconque des revendications 26 à 30, **caractérisée par le fait que** les polymères cationiques ou amphotères de densité de charge cationique ≥ 2 meq/gramme représentent en poids 0,0025% à 10%, et de préférence 0,05% à 5% du poids total de la composition.

34. Procédé de teinture des fibres kératiniques, en particulier pour fibres kératiniques humaines et plus particulièrement des cheveux, **caractérisé par le fait qu'**il consiste à appliquer sur les fibres au moins une composition colorante comprenant, dans un milieu approprié pour la teinture, au moins un colorant d'oxydation, la couleur étant révélée à pH alcalin, neutre ou acide à l'aide d'une composition oxydante comprenant, dans un milieu approprié pour la teinture, au moins un agent oxydant, qui est mélangée juste au moment de l'emploi à la composition colorante ou qui est appliquée séquentiellement sans rinçage intermédiaire, la composition colorante et la composition oxydante comprenant en outre et répartis indifféremment entre elles-deux, au moins un acide éther carboxylique polyoxyalkyléné ou l'un de ses sels, au moins un agent tensioactif non-ionique et au moins un polymère cationique ou amphotère dont la densité de charge cationique est supérieure ou égale à 2 meq/gramme, les tensioactifs non ioniques sont choisis dans le groupe formé par :
2) les alcools gras comportant de 8 à 22 atomes de carbone et oxyéthylénés par 1 à 10 moles d'oxyde d'éthylène.
2) les alcools gras mono- ou poly-glycérolés de formule (II) suivante : 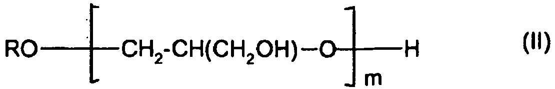 dans laquelle :
R représente un radical saturé ou insaturé, linéaire ou ramifié, comportant de 8 à 40 atomes de carbone et de préférence de 10 à 30 atomes de carbone;
m représente un nombre allant de 1 à 30 et de préférence de 1 à 10.

35. Procédé de teinture des fibres kératiniques, en particulier pour fibres kératiniques humaines et plus particulièrement des cheveux, **caractérisé par le fait qu'**il consiste à appliquer sur les fibres au moins une composition colorante comprenant, dans un milieu approprié pour la teinture, au moins un colorant d'oxydation, la couleur étant révélée à pH alcalin, neutre ou acide à l'aide d'une composition oxydante comprenant, dans un milieu approprié pour la teinture, au moins un agent oxydant, qui est mélangée juste au moment de l'emploi à la composition colorante ou qui est appliquée séquentiellement sans rinçage intermédiaire, la composition colorante ou la composition oxydante comprenant en outre et réunis dans la même composition, au moins un acide éther carboxylique polyoxyalkyléné ou l'un de ses sels, au moins un agent tensioactif non-ionique et au moins un polymère cationique ou amphotère dont la densité de charge cationique est supérieure ou égale à 2 meq/gramme, les tensioactifs non ioniques sont choisis dans le groupe formé par :
3) les alcools gras comportant de 8 à 22 atomes de carbone et oxyéthylénés par 1 à 10 moles d'oxyde d'éthylène.
2) les alcools gras mono- ou poly-glycérolés de formule (II) suivante: 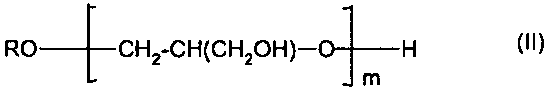 dans laquelle :
R représente un radical saturé ou insaturé, linéaire ou ramifié, comportant de 8 à 40 atomes de carbone et de préférence de 10 à 30 atomes de carbone ;
m représente un nombre allant de 1 à 30 et de préférence de 1 à 10.

36. Procédé selon la revendication 34 ou 35, **caractérisé par le fait qu'**il consiste à appliquer sur les fibres kératiniques sèches ou humides, la composition prête à l'emploi, réalisée extemporanément au moment de l'emploi à partir des compositions colorante et oxydante, à la laisser agir pendant un temps de pause variant de 1 à 60 minutes environ, et de préférence de 10 à 45 minutes, à rincer les fibres, puis éventuellement à les laver au shampooing, puis à les rincer à nouveau, et à les sécher.

37. Dispositif à plusieurs compartiments ou « Kit » pour la teinture des fibres kératiniques humaines et plus particulièrement des cheveux, **caractérisé par le fait qu'**au moins un compartiment renferme une composition colorante comprenant, dans un milieu approprié pour la teinture, au moins un colorant d'oxydation, et qu'un autre compartiment renferme une composition oxydante comprenant, dans un milieu approprié pour la teinture, un agent oxydant, la composition colorante et la composition oxydante comprenant en outre et répartis indifféremment entre elles-deux, au moins un acide éther carboxylique polyoxyalkyléné ou l'un de ses sels, au moins un agent tensioactif non-ionique et au moins un polymère cationique ou amphotère dont la densité de charge cationique est supérieure ou égale à 2 meq/gramme, les tensioactifs non ioniques sont choisis dans le groupe formé par :
4) les alcools gras comportant de 8 à 22 atomes de carbone et oxyéthylénés par 1 à 10 moles d'oxyde d'éthylène.
2) les alcools gras mono- ou poly-glycérolés de formule (II) suivante : 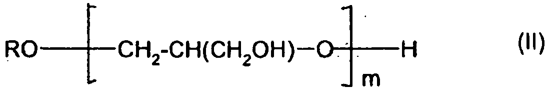 dans laquelle :
R représente un radical saturé ou insaturé, linéaire ou ramifié, comportant de 8 à 40 atomes de carbone et de préférence de 10 à 30 atomes de carbone;
m représente un nombre allant de 1 à 30 et de préférence de 1 à 10.

38. Dispositif à plusieurs compartiments ou « Kit » pour la teinture des fibres kératiniques humaines et plus particulièrement des cheveux, **caractérisé par le fait qu'**au moins un compartiment renferme une composition colorante comprenant, dans un milieu approprié pour la teinture, au moins un colorant d'oxydation, et qu'un autre compartiment renferme une composition oxydante comprenant, dans un milieu approprié pour la teinture, un agent oxydant, la composition colorante ou la composition oxydante comprenant en outre et réunis dans la même composition, au moins un acide éther carboxylique polyoxyalkyléné ou l'un de ses sels, au moins un agent tensioactif non-ionique et au moins un polymère cationique ou amphotère dont la densité de charge cationique est supérieure ou égale à 2 meq/gramme, les tensioactifs non ioniques sont choisis dans le groupe formé par :
5) les alcools gras comportant de 8 à 22 atomes de carbone et oxyéthylénés par 1 à 10 moles d'oxyde d'éthylène.
2) les alcools gras mono- ou poly-glycérolés de formule (II) suivante : 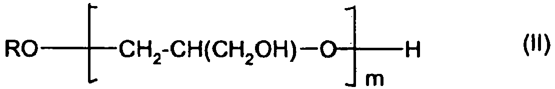 dans laquelle :
R représente un radical saturé ou insaturé, linéaire ou ramifié, comportant de 8 à 40 atomes de carbone et de préférence de 10 à 30 atomes de carbone ;
m représente un nombre allant de 1 à 30 et de préférence de 1 à 10.

## Patentansprüche

1. Zusammensetzung zum oxidativen Färben von Keratinfasern, insbesondere menschlichen Keratinfasern und besonders Haaren, die in einem zum Färben geeigneten Medium mindestens einen Oxidationsfarbstoff enthält, **dadurch gekennzeichnet, dass** sie ferner mindestens eine polyalkoxylierte Ethercarbonsäure oder eines ihrer Salze, mindestens einen nichtionischen grenzflächenaktiven Stoff und mindestens ein kationisches oder amphoteres Polymer enthält, dessen kationische Ladungsdichte größer oder gleich 2 meq/g ist, wobei
- die polyalkoxylierten Ethercarbonsäuren oder ihre Salze die folgende Formel (I) aufweisen: 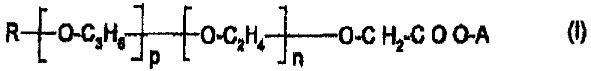 worin bedeuten:
R eine geradkettige oder verzweigte Alkyl- oder Alkenylgruppe mit 8 bis 22 Kohlenstoffatomen oder ein Gemisch von geradkettigen oder verzweigten Alkyl- oder Alkenylgruppen mit 8 bis 22 Kohlenstoffatomen,
n eine ganze Zahl oder Dezimalzahl von 2 bis 24,
p eine ganze Zahl oder Dezimalzahl von 0 bis 6,
A ein Wasserstoffatom oder Na, K, Li, 1/2 Mg oder einen Monoethanolaminrest, Ammoniumrest oder Triethanolaminrest,
- die nichtionischen grenzflächenaktiven Stoffe ausgewählt sind unter:
1) Fettalkoholen mit 8 bis 22 Kohlenstoffatomen, die mit 1 bis 10 mol Ethylenoxid ethoxyliert sind,
2) ein- oder mehrfach mit Glycerin veretherten Fettalkoholen der folgenden Formel (II): 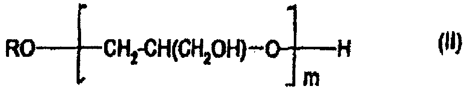 worin bedeuten:
R eine geradkettige oder verzweigte, gesättigte oder ungesättigte Gruppe mit 8 bis 40 Kohlenstoffatomen und vorzugsweise 10 bis 30 Kohlenstoffatomen;
m eine Zahl von 1 bis 30 und vorzugsweise 1 bis 10.

2. Zusammensetzung nach Anspruch 1, **dadurch gekennzeichnet, dass** in der Formel (I) R eine Alkyl(C₁₂₎gruppe bedeutet, A ein Wasserstoffatom oder Natrium ist, p = 0 und n im Bereich von 2 bis 10 liegt.

3. Zusammensetzung nach Anspruch 1, **dadurch gekennzeichnet, dass** die ein- oder mehrfach mit Glycerin veretherten Fettalkohole der Formel (II) unter dem Alkohol mit C₈/C₂₀ und 1 mol Glycerin, dem Alkohol mit C₁₀/C₁₂ und 1 mol Glycerin und dem Alkohol mit C₁₂ und 1,5 mol Glycerin ausgewählt sind.

4. Zusammensetzung nach einem der vorhergehenden Ansprüche, **dadurch gekennzeichnet, dass** die kationischen oder amphoteren Polymere mit einer kationischen Ladungsdichte von mindestens 2 meq/g ausgewählt sind unter:
1)- quartären Polymeren von Vinylpyrrolidon und Vinylimidazol
2)- Homopolymeren von Dimethyldiallylammoniumchlorid
3)- Copolymeren von Dimethyldiallylammoniumchlorid und Acrylamid
4)- Copolymeren von Acrylsäure und Dimethyldiallylammonium
5)- Polymeren von quartärem Diammonium, die aus Einheiten der folgenden Formel (V) bestehen: 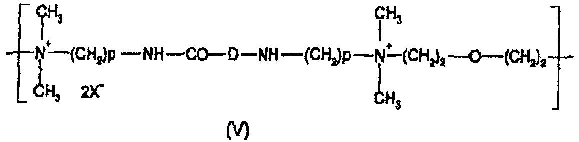 wobei in der Formel bedeuten:
p ist eine ganze Zahl von etwa 1 bis 6,
D kann nicht vorhanden sein oder eine Gruppe -(CH₂)ᵣ-CO-bedeuten, wobei r eine Zahl gleich 4 oder 7 ist, und
X⁻ ist ein Anion
6)- quartären Diammoniumpolymeren, die Wiederholungseinheiten der folgenden Formel (IV)bis enthalten: 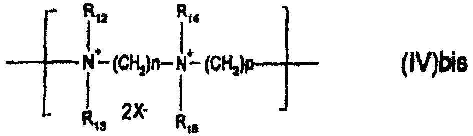 worin die Gruppen R₁₂, R₁₃, R₁₄ und R₁₅, die gleich oder verschieden sind, eine Alkyl- oder Hydroxyalkylgruppe mit etwa 1 bis 4 Kohlenstoffatomen bedeuten, n und p ganze Zahlen von 2 bis 20 sind.

5. Zusammensetzung nach Anspruch 4, **dadurch gekennzeichnet, dass** in der Formel (V) der Polymere aus Absatz (5) p 3 ist und
a) D die Gruppe -(CH₂)₄-CO- bedeutet, X ein Chloratom ist,
b) D die Gruppe - (CH₂)₇-CO- bedeutet, X ein Chloratom ist,
c) D nicht vorhanden ist, X ein Chloratom ist,
d) ein "Blockcopolymer", das aus Einheiten gebildet ist, die den unter a) und c) beschriebenen Polymeren entsprechen.

6. Zusammensetzung nach Anspruch 4, **dadurch gekennzeichnet, dass** in der Formel (IV)bis aus Absatz 6 die Gruppen R₁₂, R₁₃, R₁₄ und R₁₅ eine Methylgruppe bedeuten und n = 3, p = 6 und X = Cl oder R₁₂ und R₁₃ eine Methylgruppe bedeuten, R₁₄ und R₁₅ eine Ethylgruppe bedeuten und n = p = 3 und X = Br.

7. Zusammensetzung nach einem der vorhergehenden Ansprüche, **dadurch gekennzeichnet, dass** die alkoxylierten Ethercarbonsäuren oder ihre Salze 2 bis 15 Gew.-% und vorzugsweise 3 bis 10 Gew.-% des Gesamtgewichts der Zusammensetzung ausmachen.

8. Zusammensetzung nach einem der vorhergehenden Ansprüche, **dadurch gekennzeichnet, dass** die nichtionischen grenzflächenaktiven Stoffe 2 bis 40 Gew.-% und vorzugsweise 4 bis 20 Gew.-% des Gesamtgewichts der Zusammensetzung ausmachen.

9. Zusammensetzung nach einem der vorhergehenden Ansprüche, **dadurch gekennzeichnet, dass** die kationischen oder amphoteren Polymere mit einer kationischen Ladungsdichte ≥ 2 meq/g 0,01 bis 10 Gew.-% und vorzugsweise 0,2 bis 5 % des Gesamtgewichts der Zusammensetzung ausmachen.

10. Zusammensetzung nach einem der vorhergehenden Ansprüche, **dadurch gekennzeichnet, dass** der Oxidationsfarbstoff unter den Oxidationsbasen und Kupplern ausgewählt ist.

11. Zusammensetzung nach Anspruch 10, **dadurch gekennzeichnet, dass** sie mindestens eine Oxidationsbase enthält.

12. Zusammensetzung nach Anspruch 10 oder 11, **dadurch gekennzeichnet, dass** die Oxidationsbasen unter den ortho- oder para-Phenylendiaminen, Doppelbasen, ortho- oder para-Aminophenolen und heterocyclischen Basen sowie den Additionssalzen dieser Verbindungen mit einer Säure ausgewählt sind.

13. Zusammensetzung nach Anspruch 12, **dadurch gekennzeichnet, dass** die para-Phenylendiamine unter den Verbindungen der folgenden Formel (VII) ausgewählt sind: 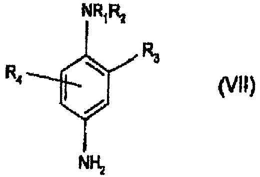 worin bedeuten:
R₁ ein Wasserstoffatom, Alkyl(C₁-₄), Monohydroxyalkyl(C₁-₄), Polyhydroxyalkyl(C₂₋₄), Alkoxy(C₁₋₄)alkyl(C₁₋₄), eine Alkyl(C₁₋₄)-gruppe, die mit einer stickstoffhaltigen Gruppe substituiert ist, Phenyl oder 4'-Aminophenyl;
R₂ ein Wasserstoffatom, Alkyl(C₁₋₄), Monohydroxyalkyl(C₁₋₄), Polyhydroxyalkyl(C₂₋₄), Alkoxy(C₁₋₄)alkyl(C₁₋₄) oder eine Alkyl(C₁₋₄)-gruppe, die mit einer stickstoffhaltigen Gruppe substituiert ist; R₁ und R₂ können auch mit dem Stickstoffatom, an das sie gebunden sind, einen 5- oder 6-gliedrigen, stickstoffhaltigen Heterocyclus bilden, der gegebenenfalls mit einer oder mehreren Gruppen Alkyl, Hydroxy oder Ureido substituiert ist;
R₃ ein Wasserstoffatom, ein Halogenatom, Alkyl(C₁₋₄), Sulfo, Carboxy, Monohydroxyalkyl(C₁₋₄), Hydroxyalkoxy(C_{1- 4}), Acetylaminoalkoxy(C₁₋₄), Mesylaminoalkoxy(C₁₋₄) oder Carbamoylaminoalkoxy(C₁₋₄);
R₄ ein Wasserstoffatom, ein Halogenatom oder Alkyl(C₁₋₄).

14. Zusammensetzung nach Anspruch 12, **dadurch gekennzeichnet, dass** die Doppelbasen unter den Verbindungen der folgenden Struktur (VIII) ausgewählt sind: 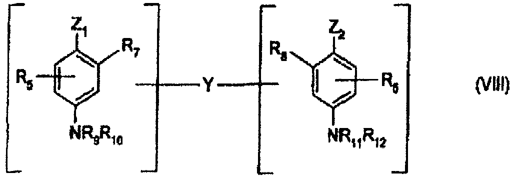 worin bedeuten:
- Z₁ und Z₂, die gleich oder verschieden sind, eine Hydroxygruppe oder eine Gruppe -NH₂, die mit einer C₁₋₄-Alkylgruppe oder einer Verbindungsgruppe Y substituiert sein können;
- die Verbindungsgruppe Y eine geradkettige oder verzweigte Alkylenkette mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen sein kann und gegebenenfalls mit einer oder mehreren Hydroxygruppen oder C₁₋₆-Alkoxygruppen substituiert ist;
- R₅ und R₆ ein Wasserstoffatom, ein Halogenatom, Alkyl(C₁₋₄), Monohydroxyalkyl(C₁₋₄), Polyhydroxyalkyl(C₂₋₄), Aminoalkyl(C₁₋₄) oder eine Verbindungsgruppe Y;
- R₇, R₈, R₉, R₁₀, R₁₁ und R₁₂, die gleich oder verschieden sind, ein Wasserstoffatom, eine Verbindungsgruppe Y oder Alkyl(C₁₋₄);
mit der Maßgabe, dass die Verbindungen der Formel (VIII) nur eine Verbindungsgruppe Y pro Molekül enthalten.

15. Zusammensetzung nach den Ansprüchen 13 oder 14, **dadurch gekennzeichnet, dass** die stickstoffhaltigen Gruppen unter den Gruppen Amino, Monoalkyl(C₁₋₄)amino, Dialkyl(C₁₋₄)amino, Trialkyl (C₁₋₄) amino, Monohydroxyalkyl(C₁₋₄)amino, Imidazolinium und Ammonium ausgewählt sind.

16. Zusammensetzung nach Anspruch 12, **dadurch gekennzeichnet, dass** die para-Aminophenole unter den Verbindungen der folgenden Struktur (IX) ausgewählt sind: 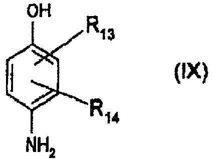 worin bedeuten:
R₁₃ ein Wasserstoffatom, ein Halogenatom, Alkyl(C₁₋₄), Monohydroxyalkyl(C₁₋₄), Alkoxy(C₁₋₄) alkyl (C₁₋₄), Aminoalkyl(C₁₋₄) oder Hydroxyalkyl(C₁₋₄)aminoalkyl(C₁₋₄),
R₁₄ ein Wasserstoffatom, ein Halogenatom, Alkyl(C₁₋₄), Monohydroxyalkyl(C₁₋₄), Polyhydroxyalkyl(C₁₋₄), Aminoalkyl (C₁₋₄), Cyanoalkyl(C₁₋₄) oder Alkoxy(C₁₋₄) alkyl (C₁₋₄).

17. Zusammensetzung nach Anspruch 12, **dadurch gekennzeichnet, dass** die heterocyclischen Basen unter den Pyridinderivaten, Pyrimidinderivaten und darunter Pyrazolopyrimidinen und Pyrazolderivaten ausgewählt sind.

18. Zusammensetzung nach den Ansprüchen 10 bis 17, **dadurch gekennzeichnet, dass** die Oxidationsbasen in Konzentrationen von 0,0005 bis 12 % und vorzugsweise 0,005 bis 8 Gew.-%, bezogen auf das Gesamtgewicht der Zusammensetzung, enthalten sind.

19. Zusammensetzung nach Anspruch 10, **dadurch gekennzeichnet, dass** die Kuppler unter den meta-Phenylendiaminen, meta-Aminophenolen, meta-Diphenolen, heterocyclischen Kupplern und den Additionssalzen dieser Verbindungen mit einer Säure ausgewählt sind.

20. Zusammensetzung nach den Ansprüchen 10 oder 19, **dadurch gekennzeichnet, dass** die Kuppler in Konzentrationen von 0,0001 bis 10 % und vorzugsweise 0,005 bis 5 Gew.-%, bezogen auf das Gesamtgewicht der Zusammensetzung, enthalten sind.

21. Zusammensetzung nach den Ansprüchen 12 oder 19, **dadurch gekennzeichnet, dass** die Additionssalze der Oxidationsbasen und Kuppler mit einer Säure unter den Hydrochloriden, Hydrobromiden, Sulfaten, Tartraten, Lactaten und Acetaten ausgewählt sind.

22. Zusammensetzung nach einem der vorhergehenden Ansprüche, **dadurch gekennzeichnet, dass** sie ferner Direktfarbstoffe enthält.

23. Zusammensetzung nach einem der vorhergehenden Ansprüche, **dadurch gekennzeichnet, dass** sie ferner mindestens ein Reduktionsmittel in Mengenanteilen von 0,05 bis 3 Gew.-%, bezogen auf das Gesamtgewicht der Zusammensetzung, enthält.

24. Zusammensetzung nach einem der vorhergehenden Ansprüche, **dadurch gekennzeichnet, dass** sie mindestens einen ergänzenden Fettalkohol in einem Gewichtsanteil von 0,001 bis 20 % des Gesamtgewichts der Zusammensetzung enthält.

25. Zusammensetzung nach einem der vorhergehenden Ansprüche, **dadurch gekennzeichnet, dass** sie mindestens ein assoziatives Polymer in einem Mengenanteil von 0,01 bis 10 % des Gesamtgewichts der Zusammensetzung enthält.

26. Gebrauchsfertige Zusammensetzung zum oxidativen Färben von Keratinfasern, insbesondere menschlichen Keratinfasern, wie Haaren, **dadurch gekennzeichnet, dass** sie durch Mischen einer Farbmittelzusammensetzung nach einem der Ansprüche 1 bis 25 und einer oxidierenden Zusammensetzung, die mindestens ein Oxidationsmittel enthält, gebildet wird.

27. Zusammensetzung nach Anspruch 26, **dadurch gekennzeichnet, dass** das Oxidationsmittel unter Wasserstoffperoxid, Harnstoffperoxid, Alkalimetallbromaten, Alkalimetallferricyaniden, Salzen von Persäuren, Redoxenzymen, wie Laccasen, Peroxidasen und Oxidoreductasen (2 Elektronen) gegebenenfalls in Gegenwart ihres Donors oder jeweiligen Cofaktors ausgewählt ist.

28. Zusammensetzung nach Anspruch 27, **dadurch gekennzeichnet, dass** das Oxidationsmittel Wasserstoffperoxid ist.

29. Zusammensetzung nach Anspruch 28, **dadurch gekennzeichnet, dass** das Oxidationsmittel eine Wasserstoffperoxidlösung mit einem Titer im Bereich von 1 bis 40 Volumina ist.

30. Zusammensetzung nach einem der vorhergehenden Ansprüche, **dadurch gekennzeichnet, dass** sie einen pH-Wert von 3 bis 12 aufweist.

31. Zusammensetzung nach einem der Ansprüche 26 bis 30, **dadurch gekennzeichnet, dass** die alkoxylierten Ethercarbonsäuren oder ihre Salze 0,5 bis 15 Gew.-% und vorzugsweise 0,7 bis 10 % des Gesamtgewichts der Zusammensetzung ausmachen.

32. Zusammensetzung nach einem der Ansprüche 26 bis 30, **dadurch gekennzeichnet, dass** die nichtionischen grenzflächenaktiven Stoffe 0,5 bis 40 Gew.-% und vorzugsweise 1 bis 20 Gew.-% des Gesamtgewichts der Zusammensetzung ausmachen.

33. Zusammensetzung nach einem der Ansprüche 26 bis 30, **dadurch gekennzeichnet, dass** die kationischen oder amphoteren Polymere mit einer kationischen Ladungsdichte ≥ 2 meq/g 0,0025 bis 10 Gew.-% und vorzugsweise 0,05 bis 5 Gew.-% des Gesamtgewichts der Zusammensetzung ausmachen.

34. Verfahren zum Färben von Keratinfasern und insbesondere menschlichen Keratinfasern, besonders zum Färben der Haare, **dadurch gekennzeichnet, dass** es darin besteht, auf die Fasern mindestens eine Farbmittelzusammensetzung aufzutragen, die in einem zum Färben geeigneten Medium mindestens einen Oxidationsfarbstoff enthält, wobei die Farbe bei einem alkalischen, neutralen oder sauren pH-Wert mit einer oxidierenden Zusammensetzung gebildet wird, die in einem zum Färben geeigneten Medium mindestens ein Oxidationsmittel enthält und die bei der Anwendung mit der Farbmittelzusammensetzung vermischt wird oder die ohne zwischenzeitliches Spülen getrennt davon anschließend aufgetragen wird, wobei die Farbmittelzusammensetzung und die oxidierende Zusammensetzung ferner beliebig zwischen ihnen verteilt mindestens eine polyalkoxylierte Ethercarbonsäure oder eines ihrer Salze, mindestens einen nichtionischen grenzflächenaktiven Stoff und mindestens ein kationisches oder amphoteres Polymer enthalten, dessen kationische Ladungsdichte größer oder gleich 2 meq/g ist, wobei die nichtionischen grenzflächenaktiven Stoffe ausgewählt sind unter:
1) Fettalkoholen mit 8 bis 22 Kohlenstoffatomen, die mit 1 bis 10 mol Ethylenoxid ethoxyliert sind,
2) ein- oder mehrfach mit Glycerin veretherten Fettalkoholen der folgenden Formel (II): 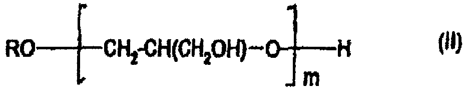 worin bedeuten:
R eine geradkettige oder verzweigte, gesättigte oder ungesättigte Gruppe mit 8 bis 40 Kohlenstoffatomen und vorzugsweise 10 bis 30 Kohlenstoffatomen;
m eine Zahl von 1 bis 30 und vorzugsweise 1 bis 10.

35. Verfahren zum Färben von Keratinfasern und insbesondere menschlichen Keratinfasern, besonders zum Färben der Haare, **dadurch gekennzeichnet, dass** es darin besteht, auf die Fasern mindestens eine Farbmittelzusammensetzung aufzutragen, die in einem zum Färben geeigneten Medium mindestens einen Oxidationsfarbstoff enthält, wobei die Farbe bei einem alkalischen, neutralen oder sauren pH-Wert mit einer oxidierenden Zusammensetzung gebildet wird, die in einem zum Färben geeigneten Medium mindestens ein Oxidationsmittel enthält und die bei der Anwendung mit der Farbmittelzusammensetzung vermischt wird oder die getrennt davon anschließend ohne zwischenzeitliches Spülen aufgetragen wird, wobei die Farbmittelzusammensetzung oder die oxidierende Zusammensetzung ferner gemeinsam in einer Zusammensetzung mindestens eine polyalkoxylierte Ethercarbonsäure oder eines ihrer Salze, mindestens einen nichtionischen grenzflächenaktiven Stoff und mindestens ein kationisches oder amphoteres Polymer enthält, dessen kationische Ladungsdichte größer oder gleich 2 meq/ g ist, wobei die nichtionischen grenzflächenaktiven Stoffe ausgewählt sind unter:
1) Fettalkoholen mit 8 bis 22 Kohlenstoffatomen, die mit 1 bis 10 mol Ethylenoxid ethoxyliert sind,
2) ein- oder mehrfach mit Glycerin veretherten Fettalkoholen der folgenden Formel (II): 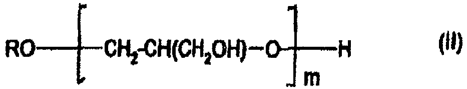 worin bedeuten:
R eine geradkettige oder verzweigte, gesättigte oder ungesättigte Gruppe mit 8 bis 40 Kohlenstoffatomen und vorzugsweise 10 bis 30 Kohlenstoffatomen;
m eine Zahl von 1 bis 30 und vorzugsweise 1 bis 10.

36. Verfahren nach Anspruch 34 oder 35, **dadurch gekennzeichnet, dass** es darin besteht, auf die trockenen oder feuchten Keratinfasern die gebrauchsfertige Zusammensetzung aufzutragen, die bedarfsgemäß bei der Anwendung aus der Farbmittelzusammensetzung und der oxidierenden Zusammensetzung gebildet wird, sie mit einer Einwirkzeit von etwa 1 bis 60 Minuten und vorzugsweise 10 bis 45 Minuten einwirken zu lassen, die Fasern zu spülen, gegebenenfalls mit Haarwaschmittel zu waschen und nochmals zu spülen und zu trocknen.

37. Vorrichtung mit mehreren Abteilungen oder "Kit" zum Färben von menschlichen Keratinfasern und besonders Haaren, **dadurch gekennzeichnet, dass** mindestens eine Abteilung eine Farbmittelzusammensetzung enthält, die in einem zum Färben geeigneten Medium mindestens einen Oxidationsfarbstoff enthält, und eine andere Abteilung eine oxidierende Zusammensetzung enthält, die in einem zum Färben geeigneten Medium ein Oxidationsmittel enthält, wobei die Farbmittelzusammensetzung und die oxidierende Zusammensetzung ferner beliebig zwischen den beiden verteilt mindestens eine alkoxylierte Ethercarbonsäure oder eines ihrer Salze, mindestens einen nichtionischen grenzflächenaktiven Stoff und mindestens ein kationisches oder amphoteres Polymer enthalten, dessen kationische Ladungsdichte größer oder gleich 2 meq/g ist, wobei die nichtionischen grenzflächenaktiven Stoffe ausgewählt sind unter:
1) Fettalkoholen mit 8 bis 22 Kohlenstoffatomen, die mit 1 bis 10 mol Ethylenoxid ethoxyliert sind,
2) ein- oder mehrfach mit Glycerin veretherten Fettalkoholen der folgenden Formel (II): 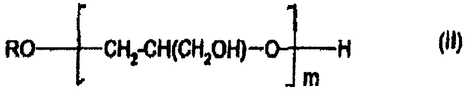 worin bedeuten:
R eine geradkettige oder verzweigte, gesättigte oder ungesättigte Gruppe mit 8 bis 40 Kohlenstoffatomen und vorzugsweise 10 bis 30 Kohlenstoffatomen;
m eine Zahl von 1 bis 30 und vorzugsweise 1 bis 10.

38. Vorrichtung mit mehreren Abteilungen oder "Kit" zum Färben von menschlichen Keratinfasern und besonders Haaren, **dadurch gekennzeichnet, dass** mindestens eine Abteilung eine Farbmittelzusammensetzung enthält, die in einem zum Färben geeigneten Medium mindestens einen Oxidationsfarbstoff enthält, und eine andere Abteilung eine oxidierende Zusammensetzung enthält, die in einem zum Färben geeigneten Medium ein Oxidationsmittel enthält, wobei die Farbmittelzusammensetzung oder die oxidierende Zusammensetzung ferner zusammen in einer Zusammensetzung mindestens eine polyalkoxylierte Ethercarbonsäure oder eines ihrer Salze, mindestens einen nichtionischen grenzflächenaktiven Stoff und mindestens ein kationisches oder amphoteres Polymer enthält, dessen kationische Ladungsdichte größer oder gleich 2 meq/g ist, wobei die nichtionischen grenzflächenaktiven Stoffe ausgewählt sind unter:
1) Fettalkoholen mit 8 bis 22 Kohlenstoffatomen, die mit 1 bis 10 mol Ethylenoxid ethoxyliert sind,
2) ein- oder mehrfach mit Glycerin veretherten Fettalkoholen der folgenden Formel (II): 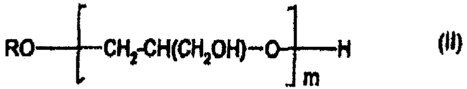 worin bedeuten:
R eine geradkettige oder verzweigte, gesättigte oder ungesättigte Gruppe mit 8 bis 40 Kohlenstoffatomen und vorzugsweise 10 bis 30 Kohlenstoffatomen;
m eine Zahl von 1 bis 30 und vorzugsweise 1 bis 10.

## Claims

1. Composition for the oxidation dyeing of keratin fibres, in particular of human keratin fibres and more particularly the hair, comprising, in a medium that is suitable for dyeing, at least one oxidation dye, **characterized in that** it also comprises at least one polyoxyalkylenated carboxylic acid ether or a salt thereof, at least one nonionic surfactant and at least one cationic or amphoteric polymer whose cationic charge density is greater than or equal to 2 meq/gram, in which
- the polyoxyalkylenated carboxylic acid ethers and the salts thereof are of formula (I) below: 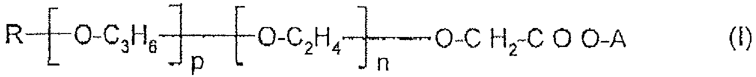 in which:
R represents a linear or branched C₈-C₂₂ alkyl or alkenyl radical or mixture of radicals,
n is an integer or fraction ranging from 2 to 24,
p is an integer or fraction ranging from 0 to 6,
A denotes a hydrogen atom or Na, K, Li, 1/2Mg or a monoethanolamine, ammonium or triethanolamine residue,
- the nonionic surfactants are chosen from the group formed by:
1) fatty alcohols containing from 8 to 22 carbon atoms and oxyethylenated with 1 to 10 mol of ethylene oxide.
2) mono- or polyglycerolated fatty alcohols of formula (II) below: 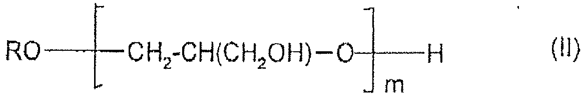 in which:
R represents a saturated or unsaturated, linear or branched radical containing from 8 to 40 carbon atoms and preferably from 10 to 30 carbon atoms;
m represents a number ranging from 1 to 30 and preferably from 1 to 10.

2. Composition according to Claim 1, **characterized in that**, in formula (I), R denotes a (C₁₂) alkyl radical, A denotes a hydrogen or sodium atom, p = 0 and n ranges from 2 to 10.

3. Composition according to Claim 1, **characterized in that** the mono- or polyglycerolated fatty alcohols of formula (II) are chosen from the C₈/C₁₀ alcohol containing one mole of glycerol, the C₁₀/C₁₂ alcohol containing 1 mol of glycerol and the C₁₂ alcohol containing 1.5 mol of glycerol.

4. Composition according to any one of the preceding claims, **characterized in that** the cationic or amphoteric polymers having a cationic charge density greater than or equal to 2 meq/gram are chosen from the group formed by:
1)- quaternary polymers of vinylpyrrolidone and of vinylimidazole
2)- homopolymers of dimethyldiallylammonium chloride
3)- copolymers of dimethyldiallylammonium chloride and acrylamide
4)- copolymers of acrylic acid and dimethyldiallylammonium
5)- quaternary diammonium polymers consisting of units of formula (V) below: 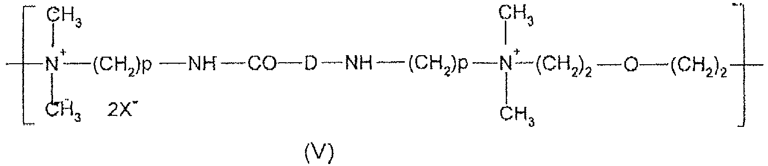 in which:
p denotes an integer varying from about 1 to 6,
D may be zero or may represent a group -(CH₂)ᵣ-CO- in which r denotes a number equal to 4 or to 7, and
X⁻ is an anion.
6)- quaternary diammonium polymers containing repeating units of formula (IV)a below: 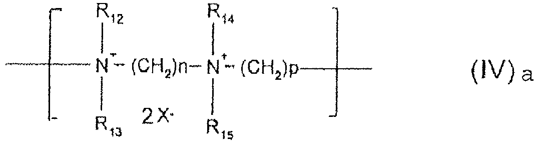 in which R₁₂, R₁₃, R₁₄ and R₁₅, which are identical or different, denote an alkyl or hydroxyalkyl radical having from about 1 to 4 carbon atoms, n and p are integers varying from 2 to 20.

5. Composition according to Claim 4, **characterized in that** in formula (V) of the polymers, paragraph (5), p is equal to 3, and,
a) D represents a group -(CH₂)₄-CO-, X denotes a chlorine atom,
b) D represents a group -(CH₂)₇-CO-, X denotes a chlorine atom,
c) D denotes the value zero, X denotes a chlorine atom,
d) a block copolymer formed of units corresponding to the polymers described in paragraphs a) and c).

6. Composition according to Claim 4, **characterized in that** in formula (IV)a, paragraph 6, R₁₂, R₁₃, R₁₄ and R₁₅ represent a methyl radical and n = 3, p = 6 and X = Cl, or alternatively R₁₂ and R₁₃ represent a methyl radical, R₁₄ and R₁₅ represent an ethyl radical and n = p = 3 and X = Br.

7. Composition according to any one of the preceding claims, **characterized in that** the oxyalkylenated carboxylic acid ethers or salts thereof represent 2% to 15% and preferably 3% to 10% by weight, relative to the total weight of the composition.

8. Composition according to any one of the preceding claims, **characterized in that** the nonionic surfactants represent 2% to 40% and preferably 4% to 20% by weight, relative to the total weight of the composition.

9. Composition according to any one of the preceding claims, **characterized in that** the cationic or amphoteric polymers having a cationic charge density ≥ 2 meq/gram represent 0.01% to 10% and preferably 0.2% to 5% by weight, relative to the total weight of the composition.

10. Composition according to any one of the preceding claims, **characterized in that** the oxidation dye is chosen from oxidation bases and couplers.

11. Composition according to Claim 10, **characterized in that** it comprises at least one oxidation base.

12. Composition according to Claim 10 or 11, **characterized in that** the oxidation bases are chosen from ortho- or para-phenylenediamines, double bases, ortho- or para-aminophenols, and heterocyclic bases, and also the addition salts of these compounds with an acid.

13. Composition according to Claim 12, **characterized in that** the para-phenylenediamines are chosen from the compounds of formula (VII) below: 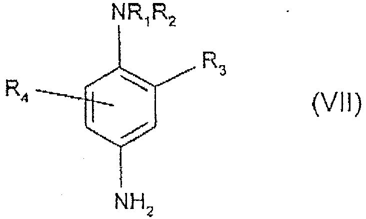 in which:
R₁ represents a hydrogen atom, a C₁-C₄ alkyl radical, a C₁-C₄ monohydroxyalkyl radical, a C₂-C₄ polyhydroxy-alkyl radical, a (C₁-C₄) alkoxy(C₁-C₄) alkyl radical or a C₁-C₄ alkyl radical substituted with a nitrogenous, phenyl or 4'-aminophenyl group;
R₂ represents a hydrogen atom, a C₁-C₄ alkyl radical, a C₁-C₄ monohydroxyalkyl radical, a C₂-C₄ polyhydroxy-alkyl radical, a (C₁-C₄)alkoxy(C₁-C₄)alkyl radical or a C₁-C₄ alkyl radical substituted with a nitrogenous group;
R₁ and R₂ may also form, with the nitrogen atom that bears them, a 5- or 6-membered nitrogen heterocycle optionally substituted with one or more alkyl, hydroxyl or ureido groups;
R₃ represents a hydrogen atom, a halogen atom, a C₁-C₄ alkyl radical, a sulfo radical, a carboxyl radical, a C₁-C₄ monohydroxyalkyl radical, a C₁-C₄ hydroxyalkoxy radical, an acetylamino(C₁-C₄)alkoxy radical, a mesylamino(C₁-C₄)alkoxy radical or a carbamoylamino(C₁-C₄)alkoxy radical,
R₄ represents a hydrogen or halogen atom or a C₁-C₄ alkyl radical.

14. Composition according to Claim 12, **characterized in that** the double bases are chosen from the compounds of structure (VIII) below: 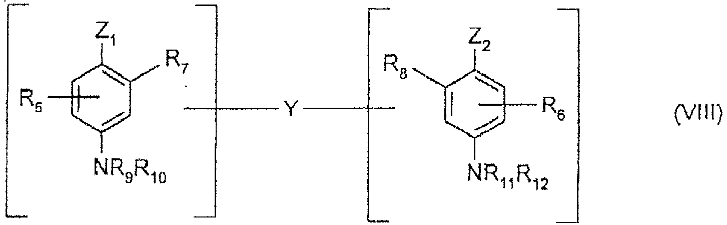 in which:
- Z₁ and Z₂, which may be identical or different, represent a hydroxyl or -NH₂ radical which may be substituted with a C₁-C₄ alkyl radical or with a linker arm Y;
- the linker arm Y represents a linear or branched alkylene chain containing from 1 to 14 carbon atoms, which may be interrupted by or terminated with one or more nitrogenous groups and/or one or more heteroatoms such as oxygen, sulfur or nitrogen atoms, and optionally substituted with one or more hydroxyl or C₁-C₆ alkoxy radicals;
- R₅ and R₆ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm Y;
- R₇, R₈, R₉, R₁₀, R₁₁ and R₁₂, which may be identical or different, represent a hydrogen atom, a linker arm Y or a C₁-C₄ alkyl radical;
it being understood that the compounds of formula (VIII) contain only one linker arm Y per molecule.

15. Composition according to Claim 13 or 14, **characterized in that** the nitrogenous groups are chosen from amino, mono (C₁-C₄)alkylamino, di(C₁-C₄)alkylamino, tri(C₁-C₄)alkylamino, monohydroxy(C₁-C₄) alkylamino, imidazolinium and ammonium radicals.

16. Composition according to Claim 12, **characterized in that** the para-aminophenols are chosen from the compounds of structure (IX) below: 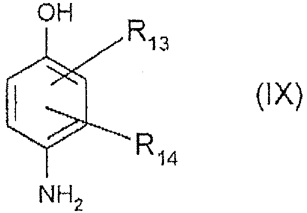 in which:
R₁₃ represents a hydrogen atom, a halogen atom, or a C₁-C₄ alkyl, C₁-C₄ monohydroxyalkyl, (C₁-C₄)alkoxy(C₁-C₄)-alkyl, C₁-C₄ aminoalkyl or hydroxy(C₁-C₄)alkylamino (C₁-C₄) alkyl radical,
R₁₄ represents a hydrogen atom, a halogen atom, or a C₁-C₄ alkyl, C₁-C₄ monohydroxyalkyl, C₂-C₄ polyhydroxyalkyl, C₁-C₄ aminoalkyl, C₁-C₄ cyanoalkyl or (C₁-C₄)alkoxy(C₁-C₄)alkyl radical.

17. Composition according to Claim 12, **characterized in that** the heterocyclic bases are chosen from pyridine derivatives, pyrimidine derivatives, including pyrazolopyrimidines, and pyrazole derivatives.

18. Composition according to Claims 10 to 17, **characterized in that** the oxidation bases are present in concentrations ranging from 0.0005% to 12% and preferably from 0.005% to 8% by weight, relative to the total weight of the composition.

19. Composition according to Claim 10, **characterized in that** the couplers are chosen from meta-phenylenediamines, meta-aminophenols, meta-diphenols and heterocyclic couplers, and the addition salts of these compounds with an acid.

20. Composition according to Claim 10 or 19, **characterized in that** the couplers are present in concentrations ranging from 0.0001% to 10% and preferably from 0.005% to 5% by weight, relative to the total weight of the composition.

21. Composition according to Claim 12 or 19, **characterized in that** the addition salts with an acid of the oxidation bases and couplers are chosen from hydrochlorides, hydrobromides, sulfates, tartrates, lactates and acetates.

22. Composition according to any one of the preceding claims, **characterized in that** it also comprises direct dyes.

23. Composition according to any one of the preceding claims, **characterized in that** it also comprises at least one reducing agent, in amounts ranging from 0.05% to 3% by weight relative to the total weight of the composition.

24. Composition according to any one of the preceding claims, **characterized in that** it comprises at least one additional fatty alcohol in a proportion by weight ranging from 0.001% to 20% relative to the total weight of the composition.

25. Composition according to any one of the preceding claims, **characterized in that** it comprises at least one associative polymer in a proportion ranging from 0.01% to 10% relative to the total weight of the composition.

26. Ready-to-use composition for the oxidation dyeing of keratin fibres, in particular of human keratin fibres such as the hair, **characterized in that** it is obtained by mixing a dye composition as defined in any one of Claims 1 to 25 and an oxidizing composition comprising at least one oxidizing agent.

27. Composition according to Claim 26, **characterized in that** the oxidizing agent is chosen from hydrogen peroxide, urea peroxide, alkali metal bromates or ferricyanides, persalts, redox enzymes such as laccases, peroxidases and 2-electron oxidoreductases, where appropriate in the presence of the respective donor or co-factor thereof.

28. Composition according to Claim 27, **characterized in that** the oxidizing agent is hydrogen peroxide.

29. Composition according to Claim 28, **characterized in that** the oxidizing agent is an aqueous hydrogen peroxide solution whose titre ranges from 1 to 40 volumes.

30. Composition according to any one of the preceding claims, **characterized in that** it has a pH ranging from 3 to 12.

31. Composition according to any one of Claims 26 to 30, **characterized in that** the oxyalkylenated carboxylic acid ethers or salts thereof represent from 0.5% to 15% and preferably from 0.7% to 10% by weight, relative to the total weight of the composition.

32. Composition according to any one of Claims 26 to 30, **characterized in that** the nonionic surfactants represent from 0.5% to 40% and preferably from 1% to 20% by weight, relative to the total weight of the composition.

33. Composition according to any one of Claims 26 to 30, **characterized in that** the cationic or amphoteric polymers having a cationic charge density ≥ 2 meq/gram represent 0.0025% to 10% and preferably 0.05% to 5% by weight, relative to the total weight of the composition.

34. Process for dyeing keratin fibres, in particular for human keratin fibres and more particularly the hair, **characterized in that** it consists in applying to the fibres at least one dye composition comprising, in a medium that is suitable for dyeing, at least one oxidation dye, the colour being developed at alkaline, neutral or acidic pH using an oxidizing composition comprising, in a medium that is suitable for dyeing, at least one oxidizing agent, which is mixed with the dye composition just at the time of use, or which is applied sequentially without intermediate rinsing, the dye composition and the oxidizing composition also comprising, distributed indifferently between the two, at least one polyoxyalkylenated carboxylic acid ether or a salt thereof, at least one nonionic surfactant and at least one cationic or amphoteric polymer whose cationic charge density is greater than or equal to 2 meq/gram, the nonionic surfactants are chosen from the group formed by:
1) fatty alcohols containing from 8 to 22 carbon atoms and oxyethylenated with 1 to 10 mol of ethylene oxide.
2) mono- or polyglycerolated fatty alcohols of formula (II) below: 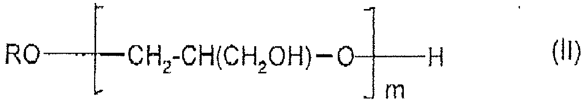 in which:
R represents a saturated or unsaturated, linear or branched radical containing from 8 to 40 carbon atoms and preferably from 10 to 30 carbon atoms;
m represents a number ranging from 1 to 30 and preferably from 1 to 10.

35. Process for dyeing keratin fibres, in particular for human keratin fibres and more particularly the hair, **characterized in that** it consists in applying to the fibres at least one dye composition comprising, in a medium that is suitable for dyeing, at least one oxidation dye, the colour being developed at alkaline, neutral or acidic pH using an oxidizing composition comprising, in a medium that is suitable for dyeing, at least one oxidizing agent, which is mixed with the dye composition just at the time of use, or which is applied sequentially without intermediate rinsing, the dye composition or the oxidizing composition also comprising, combined in the same composition, at least one polyoxyalkylenated carboxylic acid ether or a salt thereof, at least one nonionic surfactant and at least one cationic or amphoteric polymer whose cationic charge density is greater than or equal to 2 meq/gram, the nonionic surfactants are chosen from the group formed by:
1) fatty alcohols containing from 8 to 22 carbon atoms and oxyethylenated with 1 to 10 mol of ethylene oxide.
2) mono- or polyglycerolated fatty alcohols of formula (II) below: 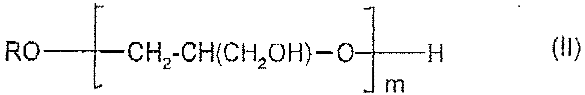 in which:
R represents a saturated or unsaturated, linear or branched radical containing from 8 to 40 carbon atoms and preferably from 10 to 30 carbon atoms;
m represents a number ranging from 1 to 30 and preferably from 1 to 10.

36. Process according to Claim 34 or 35, **characterized in that** it consists in applying the ready-to-use composition, prepared extemporaneously at the time of use from the dye composition and the oxidizing composition, to wet or dry keratin fibres, in leaving the composition to act for an exposure time ranging from 1 to 60 minutes approximately, and preferably from 10 to 45 minutes, in rinsing the fibres and then in optionally washing them with shampoo, then rinsing them again and drying them.

37. Multi-compartment device or "kit" for dyeing human keratin fibres and more particularly the hair, **characterized in that** at least one compartment contains a dye composition comprising, in a medium that is suitable for dyeing, at least one oxidation dye, and **in that** another compartment contains an oxidizing composition comprising, in a medium that is suitable for dyeing, an oxidizing agent, the dye composition and the oxidizing composition also comprising, distributed indifferently between the two, at least one polyoxyalkylenated carboxylic acid ether or a salt thereof, at least one nonionic surfactant and at least one cationic or amphoteric polymer whose cationic charge density is greater than or equal to 2 meq/gram, the nonionic surfactants are chosen from the group formed by:
1) fatty alcohols containing from 8 to 22 carbon atoms and oxyethylenated with 1 to 10 mol of ethylene oxide.
2) mono- or polyglycerolated fatty alcohols of formula (II) below: 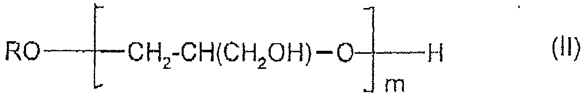 in which:
R represents a saturated or unsaturated, linear or branched radical containing from 8 to 40 carbon atoms and preferably from 10 to 30 carbon atoms;
m represents a number ranging from 1 to 30 and preferably from 1 to 10.

38. Multi-compartment device or "kit" for dyeing human keratin fibres and more particularly the hair, **characterized in that** at least one compartment contains a dye composition comprising, in a medium that is suitable for dyeing, at least one oxidation dye, and **in that** another compartment contains an oxidizing composition comprising, in a medium that is suitable for dyeing, an oxidizing agent, the dye composition or the oxidizing composition also comprising, combined in the same composition, at least one polyoxyalkylenated carboxylic acid ether or a salt thereof, at least one nonionic surfactant and at least one cationic or amphoteric polymer whose cationic charge density is greater than or equal to 2 meq/gram, the nonionic surfactants are chosen from the group formed by:
1) fatty alcohols containing from 8 to 22 carbon atoms and oxyethylenated with 1 to 10 mol of ethylene oxide.
2) mono- or polyglycerolated fatty alcohols of formula (II) below: 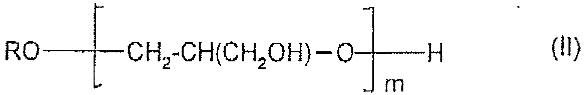 in which:
R represents a saturated or unsaturated, linear or branched radical containing from 8 to 40 carbon atoms and preferably from 10 to 30 carbon atoms;
m represents a number ranging from 1 to 30 and preferably from 1 to 10.
